(19)

Europäisches
Patentamt
European
Patent Office
Office européen
des brevets

(11) **EP 2 908 134 A1**

(12) **EUROPEAN PATENT APPLICATION**
published in accordance with Art. 153(4) EPC

(43) Date of publication:
**19.08.2015 Bulletin 2015/34**

(51) Int Cl.:
***G01N 33/53*** *(2006.01)*

(21) Application number: **13846893.9**

(22) Date of filing: **03.10.2013**

(86) International application number:
**PCT/JP2013/076918**

(87) International publication number:
**WO 2014/061456 (24.04.2014 Gazette 2014/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **15.10.2012 JP 2012228417**

(71) Applicant: **National University Corporation
Nagoya University
Aichi 464-8601 (JP)**

(72) Inventors:
• **OZAKI Norio**
**Nagoya-shi
Aichi 464-8601 (JP)**
• **NAGAI Taku**
**Nagoya-shi
Aichi 464-8601 (JP)**

• **YOSHIMI Akira**
**Nagoya-shi
Aichi 464-8601 (JP)**
• **YAMADA Shinnosuke**
**Nagoya-shi
Aichi 464-8601 (JP)**
• **HIRAKAWA Akihiro**
**Nagoya-shi
Aichi 464-8601 (JP)**
• **KUNIMOTO Shohko**
**Nagoya-shi
Aichi 464-8601 (JP)**
• **MATSUMOTO Yurie**
**Nagoya-shi
Aichi 464-8601 (JP)**

(74) Representative: **Steinecke, Peter
Müller Fottner Steinecke
Rechtsanwalts- und Patentanwaltspartnerschaft
mbB
P.O. Box 1140
52412 Jülich (DE)**

(54) **INTEGRATION DYSFUNCTION SYNDROME MARKER SET AND UTILIZATION THEREOF**

(57)    The object is to provide a combination of biomarkers which allow highly accurate discrimination of schizophrenia, and a utilization thereof. A combination of schizophrenia marker set including a combination of two or more protein molecules selected from the group consisting of trifunctional purine biosynthetic protein adenosine-3, uroporphyrinogen decarboxylase, interferon-induced GTP-binding protein Mx1, glutaredoxin-3, microtubule-associated protein RP/EB family member 1, tubulin folding cofactor B, immunoglobulin mu chain C region, and heat shock 70 kDa protein 4L is provided. In addition, a method for examining schizophrenia using the level of the marker set in a specimen as an index is provided.

**EP 2 908 134 A1**

**Description**

[TECHNICAL FIELD]

The present invention relates to a schizophrenia marker useful for the examination of schizophrenia, and more specifically to a combination of biomarkers (biomarker set) usable for the determination of onset possibility (prevalence rate) of schizophrenia, and a method for examining schizophrenia using the same. The present application claims priority based on Japanese Patent Application No. 2012-228417 filed on October 15, 2012, and the content of the patent application is hereby incorporated by reference in its entirety.

[BACKGROUND ART]

Schizophrenia is a chronic and progressive mental disorder developed during puberty and adolescence, and its main symptoms are positive symptoms (hallucinations and delusion, disorganized speech/behavior), negative symptoms (for example, flat affect, poverty of thought content, and loss of motivation) and cognition disorder (attentional dysfunction, poor working memory, and executive function deficits). Schizophrenia is regarded as a multifactorial disorder involved with genetic and environmental factors, and there are still many unknown points regarding the molecular biological mechanism of the disease state of schizophrenia, and thus no biochemical test method has been found.

Diagnosis of schizophrenia is carried out by diganostics (symptomatology) based on medical health questionnaire and patient's complaint, and there is no established diagnosis method by more empirical methods such as biochemical examination or similar. Schizophrenia progresses in several years after development, irreversible damage is caused, which likely causes permanent disturbance in social function. However, therapeutic intervention is delayed because there is no examination method useful for diagnosis, so that considerable cases are advancing in severity. Development of an examination method useful for the diagnosis of schizophrenia is desired. However, this disease is regarded as developed by disorders of cerebral molecules, while it is very difficult to detect the molecular pathogenesis in the brain, so that the development of the examination method is not proceeding.

From the result of studies such as twin studies, it was proved that hereditary factors are markedly involved with the development of schizophrenia. Some candidate genes which are suggested to be involved with the development of schizophrenia have been identified by gene analysis using the patients with schizophrenia and healthy subjects (for example, Non-Patent Literatures 1 to 3), but there are few clues for clarifying the association between these genes and the disease state of schizophrenia, so that the molecular mechanism developing the disease has not been elucidated. As a result of this, pathophysiology of the disease is still unknown, so that there is no established diagnosis method based on biochemical examination.

[CITATION LIST]

[PATENT DOCUMENT]

Patent Literature 1: Japanese Unexamined Patent Application Publication No. 2012-013415

[NON-PATENT DOCUMENT]

Non-Patent Literature 1: Arcos-Burgos M. et al., Am J Hum Genet 77,6 p937-44, 2005
Non-Patent Literature 2: Lewis CM. et al., Am J Hum Genet 73, 34-48, 2003
Non-Patent Literature 3: Badner JA. And Gershon ES., Mol Psychiatry 7, 405-11, 2002

[SUMMARY OF THE INVENTION]

[PROBLEM OF BE SOLVED BY INVENTION]

The present invention is intended to provide a combination of biomarkers and utilization thereof (for example, an examination method, an examination reagent, and an examination kit) which allow highly accurate discrimination of schizophrenia.

[MEANS FOR SOLVING PROBLEM]

In order to find biomarkers specific to schizophrenia, the inventors carried out researches with focus on the lymphocytes contained in the peripheral blood. As a result of this, 22 proteins which showed a significant difference between the patient and healthy subject groups in the expression level were reported (Patent Literature 1). Most of the identified proteins were found to be expressed in the brain. Therefore, the possibility of their deep involvement with pathogenesis of schizophrenia was strongly suggested.

Each of the 22 identified proteins is highly useful. However, high discrimination accuracy as possible is demanded in clinical fields, and such demand is hard to be satisfied by a single biomarker. In addition, in order to achieve further higher accuracy in the discrimination of schizophrenia or other disease developed by various factors, combination of a plurality of biomarkers is considered advantageous. From this viewpoint, the identification of the combinations of biomarkers which allow highly accurate discrimination was aimed using a statistical method. As a result of this, markedly effective biomarkers were found, and the combinations of biomarkers that bring about very high discrimination accuracy were successfully identified. The examination using the combination of the specified biomarkers is markedly practical, and is expected to contribute to dramatic improvement in diagnosis techniques of schizophrenia. The present invention described below is mainly based on the above results.

[1] A schizophrenia marker set including a combination of two or more protein molecules selected from the group consisting of trifunctional purine biosynthetic protein adenosine-3, uroporphyrinogen decarboxylase, interferon-induced GTP-binding protein Mx1, glutaredoxin-3, microtubule-associated protein RP/EB family member 1, tubulin folding cofactor B, immunoglobulin mu chain C region, and heat shock 70 kDa protein 4L.

[2] The schizophrenia marker set of [1], which includes at least trifunctional purine biosynthetic protein adenosine-3, uroporphyrinogen decarboxylase, interferon-induced GTP-binding protein Mx1, and glutaredoxin-3.

[3] The schizophrenia marker set of [2], which further includes microtubule-associated protein RP/EB family member 1 and/or tubulin folding cofactor B.

[4] The schizophrenia marker set of [1], which is a combination of trifunctional purine biosynthetic protein adenosine-3, uroporphyrinogen decarboxylase, interferon-induced GTP-binding protein Mx1, glutaredoxin-3, microtubule-associated protein RP/EB family member 1, and tubulin folding cofactor B.

[5] The schizophrenia marker set of [1], which is a combination of trifunctional purine biosynthetic protein adenosine-3, uroporphyrinogen decarboxylase, interferon-induced GTP-binding protein Mx1, and glutaredoxin-3.

[6] The schizophrenia marker set of [1], which includes at least trifunctional purine biosynthetic protein adenosine-3 and interferon-induced GTP-binding protein Mx1.

[7] The schizophrenia marker set of [1], which is a combination of trifunctional purine biosynthetic protein adenosine-3 and interferon-induced GTP-binding protein Mx1.

[8] The schizophrenia marker set of [1], which includes one or more protein molecules selected from the group consisting of trifunctional purine biosynthetic protein adenosine-3, interferon-induced GTP-binding protein Mx1, and heat shock 70 kDa protein 4L.

[9] An examination method for schizophrenia using the level of the schizophrenia marker set of any one of [1] to [8] in the specimen as an indication.

[10] The examination method for schizophrenia of [9], which includes the following steps (1) to (3):

(1) a step of preparing a specimen derived from the subject;
(2) a step of detecting the protein molecules composing the marker set in the specimen; and
(3) a step of determining the present or future development possibility of schizophrenia based on the detection result.

[11] The examination method for schizophrenia of [10], wherein a lower detected value shows a higher development possibility of schizophrenia for trifunctional purine biosynthetic protein adenosine-3, uroporphyrinogen decarboxylase, glutaredoxin-3, microtubule-associated protein RP/EB family member 1, tubulin folding cofactor B, and heat shock 70 kDa protein 4L, and a higher detected value shows a higher development possibility of schizophrenia for interferon-induced GTP-binding protein Mx1 and immunoglobulin mu chain C region.

[12] The examination method for schizophrenia of [10] or [11], wherein the determination of step (3) is carried out using a logistic model.

[13] The examination method for schizophrenia of any one of [9] to [12], wherein the specimen is blood, blood plasma, blood serum, oral mucosa, nasal mucosa, skin, blood cells, or lymphoblastoid cells prepared by immortalizing the blood lymphocytes collected from the subject.

[14] A kit for examining schizophrenia including a reagent for detecting the protein molecules composing the schizophrenia marker set of [1], and an instruction manual.

[15] The kit for examining schizophrenia of [14], wherein each of the reagents is an antibody to the target protein molecule.

[BRIEF DESCRIPTION OF THE DRAWINGS]

Fig. 1 shows the result of expression analysis (Western blot test and 2D-DIGE method). The S/C (the ratio between the schizophrenia patient group and the healthy subject group) and the p value (Student's t-test) of each analysis are shown.

Fig. 2 shows the characteristics of the top 30 models with a higher corrected AUC.

Fig. 3 shows the odds ratio of protein contained in the candidate model 1.

Fig. 4 shows the odds ratio of protein contained in the candidate model 2.

Fig. 5 shows the validation of effectiveness of the selected biomarkers. The 30 specimens used for screening the biomarkers (screening data) and other 30 specimens (validation data) were used for the validation.

Fig. 6 shows the validation of the effectiveness of the selected biomarkers. The validation results are summarized together with the explanation of the functions of the biomarkers.

Fig. 7 shows the validation of the four biomarkers (MX1, GART, UROD, and GLRX3) using a validation data set. The evaluation used a four-variable model.

Fig. 8 shows the validation of the four biomarkers (MX1, GART, UROD, and GLRX3) using a validation data set. The predicting performance of the four-variable model for the validation data was evaluated. The AUC was 0.724. In consideration of the number of data (N = 30), reproducibility is high.

Fig. 9 shows the validation of the six biomarkers (MX1, GART, UROD, GLRX3, MAPRE1, and TBCB) using the validation data set. The evaluation used a six-variable model.

Fig. 10 shows the validation of the six biomarkers (MX1, GART, UROD, GLRX3, MAPRE1, and TBCB) using a validation data set. Prediction performance of the six-variable model for the validation data was evaluated. The AUC was 0.664.

Fig. 11 shows the correlation between MX1, GART, and HSPA4L (correlation coefficient for the validation data, and the correlation coefficient integrating the both data).

Fig. 12 shows various predicting models and their discrimination accuracies. The upper four predicting models constructed the predicting models based on screening data sets, and evaluated them based on a validation data set. The middle four predicting models constructed the predicting models based on validation data sets, and evaluated them based on screening data sets. The lower four predicting models constructed the predicting models based on screening and validation data sets. S: screening data set, V: validation data set, I: integration of screening and validation data sets.

[DESCRIPTION OF EMBODIMENTS]

1. Schizophrenia marker set

A first aspect of the present invention relates to a schizophrenia marker set including a combination of protein molecules (biomarkers) specific to schizophrenia (hereinafter may be referred to as "the biomarker set of the present invention"). The biomarker set of the present invention is useful for the determination and evaluation of present or future development possibility of schizophrenia. The "present development possibility" represents whether the patient is affected by or has developed schizophrenia (whether the patient has schizophrenia or not) at the time of inspection, or the probability of onset or development. On the other hand, the "future development possibility" represents future possibility (risk) of development of schizophrenia. "Schizophrenia" is a generic name of psychiatric disorders based on fragmentation of mental functions. According to the International Statistical Classification of Diseases and Related Health Problems (ICD) by the World Health Organization (WHO), the schizophrenia is classified into Paranoid schizophrenia, Hebephrenic schizophrenia, Catatonic schizophrenia, Undifferentiated schizophrenia, Post-schizophrenic depression, Residual schizophrenia, and Simple schizophrenia.

The biomarker set of the present invention is a combination of protein molecules that were found to be correlated with schizophrenia. More specifically, the biomarker set of the present invention is composed of two or more protein molecules selected from the eight proteins listed below. The biomarker set of the present invention is composed of preferably three or more, more preferably four or more protein molecules:

trifunctional purine biosynthetic protein adenosine-3 (hereinafter referred to as "GART", SEQ ID NO. 1);
uroporphyrinogen decarboxylase (hereinafter referred to as "UROD", SEQ ID NO. 2);

interferon-induced GTP-binding protein Mx1 (hereinafter referred to as "MX1", SEQ ID NO. 3);
glutaredoxin-3 (hereinafter referred to as "GLRX3", SEQ ID NO. 4);
microtubule-associated protein RP/EB family member 1 (hereinafter referred to as "MAPRE1", SEQ ID NO. 5);
tubulin folding cofactor B (hereinafter referred to as "TBCB", SEQ ID NO. 6);
immunoglobulin mu chain C region (hereinafter referred to as "IGHM", SEQ ID NO. 7); and
heat shock 70 kDa protein 4L (hereinafter referred to as "HSPA4L", SEQ ID NO. 8).

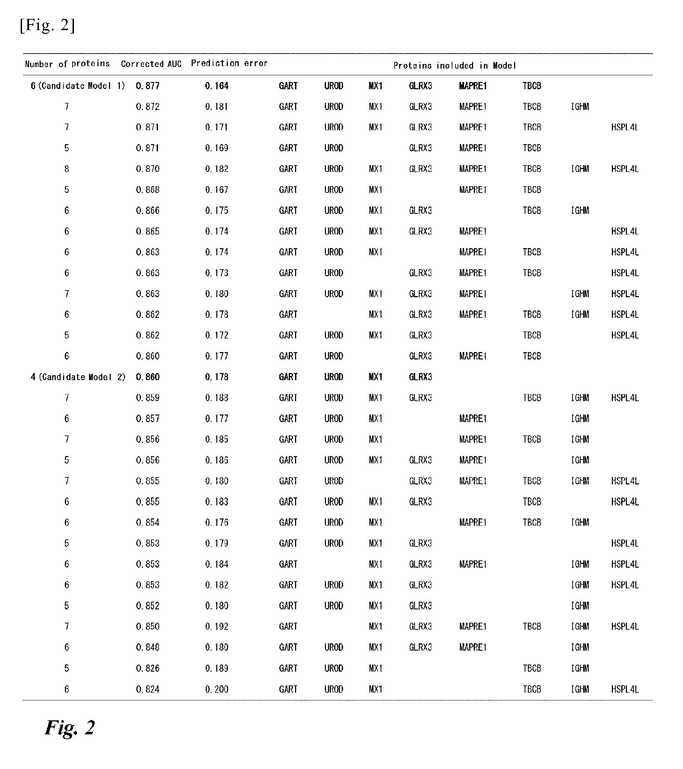

The biomarker set of the present invention may include various combinations of the above-described proteins. As shown in the below-described examples (in particular, the table of Fig. 2), GART, UROD, MX1, and GLRX3 are particularly useful and important for allowing highly accurate discrimination. Therefore, according to a preferred embodiment of the present invention, a schizophrenia marker set including at least the four protein molecules is provided. It was also shown that MAPRE1 and TBCB are useful secondary to the above-described four protein molecules. Therefore, it is also preferred that the above-described four protein molecules be combined with one or both of them to make a schizophrenia marker set.

Specific examples of the biomarker set of the present invention are listed below. They were specified as the combinations achieving high discrimination accuracy, as a result of the analysis by a statistical method:

a combination of GART, UROD, MX1, GLRX3, MAPRE1, and TBCB;
a combination of GART, UROD, MX1, GLRX3, MAPRE1, TBCB, and IGHM;
a combination of GART, UROD, MX1, GLRX3, MAPRE1, TBCB, and HSPA4L;
a combination of GART, UROD, GLRX3, MAPRE1, and TBCB;
a combination of GART, UROD, MX1, GLRX3, MAPRE1, TBCB, IGHM, and HSPA4L;
a combination of GART, UROD, MX1, MAPRE1, and TBCB;
a combination of GART, UROD, MX1, GLRX3, TBCB, and IGHM;
a combination of GART, UROD, MX1, GLRX3, MAPRE1, and HSPA4L;
a combination of GART, UROD, MX1, MAPRE1, TBCB, and HSPA4L;
a combination of GART, UROD, GLRX3, MAPRE1, TBCB, and HSPA4L;
a combination of GART, UROD, MX1, GLRX3, MAPRE1, IGHM, and HSPA4L;
a combination of GART, MX1, GLRX3, MAPRE1, TBCB, IGHM, and HSPA4L;
a combination of GART, UROD, MX1, GLRX3, TBCB, and HSPA4L;
a combination of GART, UROD, GLRX3, MAPRE1, and TBCB;
a combination of GART, UROD, MX1, and GLRX3;
a combination of GART, UROD, MX1, GLRX3, TBCB, IGHM, HSPA4L;
a combination of GART, UROD, MX1, MAPRE1, and IGHM ;
a combination of GART, UROD, MX1, MAPRE1, TBCB, and IGHM;
a combination of GART, UROD, MX1, GLRX3, MAPRE1, and IGHM;
a combination of GART, UROD, GLRX3, MAPRE1, TBCB, IGHM, and HSPA4L;
a combination of GART, UROD, MX1, GLRX3, TBCB, and HSPA4L;
a combination of GART, UROD, MX1, MAPRE1, TBCB, and IGHM;
a combination of GART, UROD, MX1, GLRX3, and HSPA4L;
a combination of GART, MX1, GLRX3, MAPRE1, IGHM, and HSPA4L;
a combination of GART, UROD, MX1, GLRX3, IGHM, and HSPA4L;
a combination of GART, UROD, MX1, GLRX3, and IGHM;
a combination of GART, MX1, GLRX3, MAPRE1, TBCB, IGHM, and HSPA4L;
a combination of GART, UROD, MX1, GLRX3, MAPRE1, and IGHM;
a combination of GART, UROD, MX1, TBCB, and IGHM; and
a combination of GART, UROD, MX1, TBCB, IGHM, and HSPA4L.

Among the above combinations, the combinations of GART, UROD, MX1, GLRX3, MAPRE1, and TBCB showed the highest discrimination accuracy. In consideration of this fact, this combination is regarded as one of the optimum biomarker sets. In addition, according to the Occam's razor, which is a principle in statistics, the number of proteins used for discrimination is preferably smaller when the discrimination accuracy is at the same level (details will be described later). The combination of GART, UROD, MX1, and GLRX3 most conforms to this principle.

As shown in the below-described examples, as a result of validation of reproducibility, it was found that GART and MX1 have good reproducibility. In view of this finding, it is preferred that the biomarker set includes GART and MX1. In particular, it is preferred that the biomarker set be composed of GART and MX1. On the other hand, in consideration of the finding that GART and MX1 have good reproducibility and the finding that HSPA4L also has relatively good reproducibility, according to a preferred embodiment of the present invention, the biomarker set includes one or more protein molecules selected from the group consisting of GART, MX1, and HSPA4L.

2. Method for examining schizophrenia

A second aspect of the present invention relates to the utilization of the biomarker set of the present invention, and provides a method for examining the present or future development possibility of schizophrenia (hereinafter may be referred to as "the examination method of the present invention"). The examination method of the present invention is useful as a means for determining whether the patient is affected by or has developed schizophrenia, or a means for determining the possibility of future development of schizophrenia. The examination method of the present invention provides information useful for diagnosing schizophrenia. The examination method of the present invention allows simple and objective determination of development possibility of schizophrenia.

The delay of time from the development of a mental disease such as schizophrenia to the initiation of drug therapy is referred to as duration of untreated psychosis (DUP). It is known that the longer the DUP, the worse the clinical outcome. On the other hand, several years from the development are the important period, which influences the prognosis, and is referred to as critical period. According to a finding of cerebral nerve image analysis, it is reported that the longer the DUP, the smaller the volume of the planum temporale, which is a part of left gyrus temporalis superior (Takahashi et al.: Psychiatry Res 154: 209-219, 2007). This finding suggests that the changes in brain morphology proceeds during the untreated duration in the early stage (critical period) of the disease, and that it is very important to shorten the DUP, as well as to early intervene to improve the neurobiological changes in the critical period. The examination method of the present invention can be used also for screening of schizophrenia, and is useful for early detection and early treatment of schizophrenia.

The examination method of the present invention uses the level of the biomarker set of the present invention in the specimen derived from the subject as an indication. The "level" herein typically means the "amount" or "concentration". However, according to the convention and technical common knowledge, the term "level" is used also for expressing whether the molecules to be detected can be detected or not (more specifically, the presence or absence of apparent presence). The biomarker set to be used may be selected in consideration of the demanded accuracy and convenience of the examination.

The examination method of the present invention carries out the following steps:

(1) a step of preparing a specimen derived from the subject;
(2) a step of detecting the protein molecules composing the marker set (more specifically, a combination of two or more (preferably three or more, more preferably four or more) protein molecules selected from the group consisting of GART, UROD, MX1, GLRX3, MAPRE1, TBCB, IGHM, and HSPA4L) in the specimen; and
(3) a step of determining the present or future development possibility of schizophrenia based on the detection result.

In the step (1), a specimen derived from a subject is prepared. Examples of the specimen include the blood, blood plasma, blood serum, oral mucosa, nasal mucosa, and skin of the subject. Alternatively, the blood cells derived from the subject may be used as the specimen. Alternatively, the lymphocytes, B-cells, or T-cells separated from the blood cells may be used as the specimen. Alternatively, the cells after subcultivation may be used. On the other hand, lymphoblastoid cells prepared by immortalizing the blood lymphocytes collected from the subject may be used as the specimen. The "lymphoblastoid cells" are the cells similar to the cells before differentiation into mature lymphocytes, and can be prepared by immortalizing the lymphocytes separated from the blood by Epstein-Barr virus. The established lymphoblastoid cells show a protein expression profile, which more highly reflects the features and characteristics of the disease in comparison with fresh blood cells. Accordingly, the use of the lymphoblastoid cells as the specimen prevents masking of the intrinsic expression profile (more specifically, the expression profile to be detected by the method of the present invention) by the conditions of the subject (condition-dependent factors) at the time of collection of the cells, which are the elements other than the disease. As a result of this, an examination result with higher reliability which more highly reflects the features and characteristics of the disease can be obtained. The examination using, for example, the blood, blood plasma, or blood serum as the specimen is easy in operation, and thus is particularly useful as a screening examination. On the other hand, the examination using the lymphoblastoid cells as the specimen gives a result with higher reliability, and thus is particularly useful as a means for secondary examination or definite diagnosis. Accordingly, highly efficient and reliable diagnosis is achieved by targeting the high-risk group (the subjects with high development possibility) by the examination using, for example, the blood, blood plasma, or blood serum as the specimen, followed by carrying out the examination using the lymphoblastoid cells as the specimen.

The subject is not particularly limited. More specifically, the present invention is widely applicable to the subjects requiring the determination of the present or future development possibility of schizophrenia (more specifically, the presence or absence of onset or development of schizophrenia, the degree of possibility of development of schizophrenia, and the degree of possibility of future development of schizophrenia). For example, when the present invention is applied to a patient who has been diagnosed to have schizophrenia by medical health questionnaire, propriety of the diagnosis can be reviewed based on an objective index, or the level of protein expression. More specifically, the examination method of the present invention provides information, which helps or supports prior art diagnosis. This information is useful for the decision of more appropriate treatment policy, and promotes the improvements in therapeutic effect and patient's QOL (Quality of Life). On the other hand, the present invention can be used to monitor the disease condition, thereby preventing the increase in obstinacy and seriousness, and recurrence.

Those presumed to have a high risk of onset of schizophrenia based on the family background and other factors (high risk subjects) are also preferred subjects. The application of the present invention to the subject before development of the symptoms of schizophrenia allows inhibition or delay of the development, or therapeutic intervention in the early stage of the disease. The present invention is also useful for identification of those having a high risk of onset of schizophrenia. This identification allows, for example, the decrease in the development possibility (possibility of acquiring disease) owing to precautions and improvements in the lifestyle habit. Those who cannot or are difficult to be determined whether they have schizophrenia or not by prior art diagnosis, such as those having no subjective symptom, are also preferred subjects in the present invention. The present invention may be carried out as a term of health examination.

In the step (2), the biomarkers in the specimen are detected. It is not essential to strictly determine the level of each biomarker. More specifically, the level of the biomarkers is detected to a degree that allows the determination of development possibility of schizophrenia in the subsequent step (3). For example, the detection may be carried out so as to allow the discrimination whether the level of the biomarkers in the specimen exceeds the predetermined criterion value or not.

The method for detecting each biomarker is not particularly limited, but preferably an immunological method is used. An immunological method allows prompt and highly sensitive detection. In addition, operation is easy. Measurement by an immunological method uses a substance having specific binding property to the biomarker to be used. The substance is usually an antibody, but may be not an antibody as long as the substance has specific binding property to the biomarker, and the amount of binding is measurable. The antibody may be not a commercially available antibody, and may be newly prepared by, for example, an immunological method, a phage display method, or a ribosome display method.

Examples of the measurement method include latex agglutination assay, fluoroimmunoassay (FIA method), enzyme immunoassay (EIA method), radioimmunoassay (RIA method), and Western blot test method. Examples of the preferred measurement method include the FIA method and EIA method (including ELISA method). These methods allow high-sensitivity, prompt, and convenient detection. The FIA method uses a fluorescent-labeled antibody, and detects an antigen-antibody complex (immune complex) using fluorescence as a signal. On the other hand, the EIA method uses an enzymatically labeled antibody, and detects an immune complex using color development or luminescence based on enzyme reaction as a signal.

The ELISA method has many advantages such as high detection sensitivity, high specificity, marked quantitative determination performance, easy operation, and suitability for simultaneous processing of multiple specimens. An example of the specific operation method using the ELISA method is described below. Firstly, an anti-biomarker antibody is immobilized on an insoluble support. Specifically, for example, the surface of a microplate is immunized (coated) with an anti-biomarker monoclonal antibody. The specimen is brought into contact with the immobilized antibody. As a result of this operation, an immune complex is formed if the antigen (protein molecule as the biomarker) to the immobilized anti-biomarker antibody is present in the specimen. After the non-specific binding component is removed by washing operation, the immune complex is labeled by adding an enzyme-bound antibody, and then color development by reaction with the enzymatic substrate is carried out. Subsequently, the immune complex is detected using the amount of color development as the index. Details about the ELISA method are described in many publications and literatures, and establishment of the experimental procedure and conditions of the methods may refer to them. A competition method (a method including addition of an antigen and a specimen for competition) may be used in place of non-competition method. Alternatively, a method directly detecting the biomarker in the specimen with a labeling antibody, or a method for sandwich method may be used. The sandwich method uses two antibodies having different epitopes (trapping antibody and detecting antibody).

A means that allows simultaneous detection of multiple specimens, such as a protein array or protein chip, may be used. As the probe, for example, an antibody specific to the target biomarker is used.

In the step (3), present or future development possibility of schizophrenia is determined based on the detection result. In order to allow highly accurate determination, the determination is preferably carried out after comparing the detected value obtained in the step (2) with the detected value of the control specimen. The determination of the development possibility may be qualitative or quantitative. As is evident from the criteria for determination, the determination herein may be carried out automatically or mechanically without depending on diagnosis by specialists such as physicians or laboratory technician.

In the present invention, in principle, the following criteria are applied to each biomarker. More specifically, for GART, UROD, GLRX3, MAPRE1, TBCB, and HSPA4L, the development possibility of schizophrenia is high when the detected value is low (the detected value and development possibility are in negative correlation with each other), and for MX1 and IGHM, the development possibility of schizophrenia is high when the detected value is high (the detected value and development possibility are in positive correlation with each other).

The determination in the step (3) may be carried out using a logistic model (statistical model) corresponding to the biomarker set to be used. A specific example (the case using the biomarker set composed of GART, UROD, MX1, GLRX3, MAPRE1, and TBCB) is described below.

<Determination using logistic model>

In the following logistic model, the p is calculated. Specifically, the level (detected value) of each protein molecule is assigned to the following formula to obtain the development possibility of schizophrenia (prevalence rate of schizophrenia).

[Formula 1]

$$p = \frac{\exp{(11.54 - 4.64 GART - 2.67 UROD + 2.04 MX1 - 3.14 GLRX3 - 1.65 MARPRE1 - 2.01 TBCB)}}{1 + \exp{(11.54 - 4.64 GART - 2.67 UROD + 2.04 MX1 - 3.14 GLRX3 - 1.65 MARPRE1 - 2.01 TBCB)}}$$

Alternatively, a criterion value or cutoff value is established, and qualitative determination such as the following (a) or (b) may be made. The criterion value or cutoff value used for determination may be decided in consideration of the type and condition of the specimen used, and demanded accuracy (confidence level).

(a) The case where all the biomarkers contained in the biomarker set are positive (cutoff value or more) is determined as positive (for example, development possibility is 50% or more), and the other case is determined as negative (for example, development possibility is less than 50%).
(b) The case where at least one of the biomarkers contained in the biomarker set is positive (cutoff value or more) is determined as positive (for example, development possibility is 50% or more), and the other case is determined as negative (for example, development possibility is less than 50%).

Alternatively, a plurality of determination categories may be provided, and subjected to quantitative or semiquantitative determination. When quantitative determination is made, for example, a plurality of determination categories related to the ranges of the level of the biomarkers (development possibility of schizophrenia is defined for each determination category) are established in advance, and the determination category of the specimen is specified from the level of the biomarkers. The number of the determination categories, and the level of the biomarkers and determination result related to the determination categories are not particularly limited, and may be freely established based on, for example, a preliminary experiment. For example, the "threshold" determining the level of the development possibility using the predetermined threshold as the border, and "the range of biomarker level" relating to the category concerning with the level of development possibility can be decided by statistical analysis using many specimens. When the analysis is carried out using statistical processing, it is commonly effective to establish high risk and low risk groups. Examples of the high-risk group include the group of patients with schizophrenia and the group of those having many family members with schizophrenia, and examples of the low risk group include the group of healthy subjects and the group without family member with schizophrenia.

According to one embodiment of the present invention, in one subject, the biomarker level measured at one point is compared with the biomarker level measured past, and the presence or absence and/or the degree of increase and decrease of each biomarker level. The data thus obtained related to the level change of the biomarker set is useful information for monitoring the change of the development possibility of schizophrenia. More specifically, based on the level variation of the biomarker set, determination whether the development possibility is increased, decreased, or not changed during the period from the former examination to the present examination can be made. If this evaluation is carried out in parallel with the treatment of schizophrenia, therapeutic effect can be confirmed, and a foreboding sign of recurrence of schizophrenia can be grasped. As a result of this, more suitable treatment policy can be decided. Accordingly, the present invention can make marked contribution to the maximization of therapeutic effect and the improvement of the QOL (quality of life) of the patient.

3. Schizophrenia examination kit

The present invention further provides a kit for examining development possibility of schizophrenia. The kit of the present invention includes reagents for detecting the protein molecules composing the schizophrenia marker set. A reagent is provided for each biomarker (protein molecule). The reagents are composed of substances showing specific binding properties to the corresponding biomarkers (hereinafter referred to as "binding molecule"). For example, a reagent for GART is the substance having specific binding properties to GART. Examples of the binding molecule include antibodies that specifically recognize biomarkers, nucleic acid aptamers, and peptide aptamers. The binding molecules are not particularly limited as to their kind and origin, as long as they have specific binding properties to the biomarkers to be used. In addition, when an antibody is used, it may be a polyclonal antibody, an oligoclonal antibody (a mixture of various antibodies), or a monoclonal antibody. The polyclonal antibody or oligoclonal antibody may be an IgG fraction derived from antiserum obtained by animal immunization, or an affinity-purified antibody by an antigen. The anti-biomarker antibody may be a fragment of an antibody such as Fab, Fab', F(ab')$_2$, scFv, or dsFv antibody.

The binding molecule may be prepared in a common procedure. If a commercial product is available, the commercial product may be used. For example, an antibody may be prepared by an immunological method, a phage display method, or a ribosome display method. The preparation of a polyclonal antibody by an immunological method may be carried out by the following procedure. An antigen (biomarker or its portion) is prepared, and an animal such as a rabbit is immunized using the antigen. An antigen is obtained by purification of a living body sample. Alternatively, a recombinant antigen may be used. The recombinant antigen can be prepared by, for example, a gene (or a portion of the gene) coding a biomarker is introduced into an appropriate host using a vector, and expressed in the recombinant cell thus obtained.

In order to enhance the immunological induction action, an antigen bound with a carrier protein may be used. Examples of the carrier protein include KLH (Keyhole Limpet Hemocyanin), BSA (Bovine Serum Albumin), and OVA (Ovalbumin). Binding between the antigen and the carrier protein can use, for example a carbodiimide method, a glutaraldehyde method, a diazo condensation method, or an MBS (maleimide benzoyloxy succinimide) method. On the other hand, an antigen prepared by expressing a biomarker molecule (or its portion) as a fused protein with GST, β-galactosidase, maltose-bound protein, or histidine (His) tag can be used. The fused protein can be purified simply by a general-purpose method.

As necessary, immunization is repeated, the blood is collected after the antibody titer is thoroughly increased, and the blood serum is obtained by, for example, centrifugation treatment. The antiserum thus obtained is subjected to affinity purification, and thus obtaining a polyclonal antibody.

On the other hand, the monoclonal antibody may be prepared by the following procedure. Firstly, immunization operation is carried out by the above-described procedure. As necessary, immunization is repeated, and antibody-producing cells are extracted from the immunized animal after the antibody titer is thoroughly increased. Secondly, the antibody-producing cells thus obtained and myeloma cells are fused to obtain hybridoma. Subsequently, the hybridoma is cloned from a single cell, and then a clone producing an antibody having high specificity to the target protein is selected. The culture solution of the selected clone is purified to obtain the desired antibody. Alternatively, the hybridoma is proliferated to the desired number or more, and then the cells are transplanted into the abdominal cavity of an animal (for example, mouse), and proliferated in the ascites fluid, and the ascites fluid is purified to obtain the desired antibody. Affinity chromatography using, for example, protein G, protein A is suitable for the purification of the above-described culture solution or ascites fluid. Alternatively, affinity chromatography including an immobilized antigen may be used. Other method such as ion exchange chromatography, gel filtration chromatography, ammonium sulfate fractionation, or centrifugation may be used. These methods may be used alone or in combination.

Providing that the specific binding properties to a biomarker are maintained, the antibody thus obtained may be subjected to various modifications. The modified antibody may be used as the reagent in the present invention.

When the specific binding molecule is a labeling antibody, the amount of bound antibody can be directly detected using the amount of labeling as the index. Accordingly, the test method is more simplified. On the other hand, there are problems that an antibody bound with a labeling agent must be prepared, and that the detection sensitivity is commonly low. Therefore, an indirect detection method such as a method using a secondary antibody bound to a labeling agent, or a method using a polymer bound to a secondary antibody and a labeling agent is preferred. The secondary antibody herein is an antibody having specific binding properties to the antibody specific to the biomarker to be used. For example, when an antibody specific to the biomarker is prepared as a rabbit antibody, an anti-rabbit IgG antibody may be used as a secondary antibody. Labeled secondary antibodies usable for various types of antibodies such as rabbit, goat, and mouse antibodies are commercially available (for example, Funakoshi Co., Ltd. and Cosmo Bio Co., Ltd.), and suitable one may be appropriately selected according to the reagent in the present invention.

Examples of the labeling agent include enzymes such as peroxidase, microperoxidase, horseradish peroxidase (HRP), alkaline phosphatase, β-D-galactosidase, glucose oxidase, and glucose-6-phosphate dehydrogenase; fluorescent substances such as fluorescein isothiocyanate (FITC), tetramethyl rhodamine isothiocyanate (TRITC), and europium; chemiluminescence substances such as luminol, isoluminol, and acridinium derivatives; coenzymes such as NAD; biotin; and radioactive materials such as $^{131}$I and [125]I.

In one embodiment, the reagent in the present invention is solid-phased according to the intended use. The insoluble support used for solidification is not particularly limited. For example, an insoluble support made of water-insoluble substance such as a resin including a polystyrene resin, a polycarbonate resin, a silicon resin, and a nylon resin, or glass. The insoluble support carries an antibody by physical adsorption or chemical adsorption.

The kit of the present invention usually includes an instruction manual. The kit may include other reagents (for example, a buffer, a blocking reagent, an enzymatic substrate, and a color-producing reagent) used when carrying out the examination method and/or an apparatus or instrument (for example, a container, a reaction apparatus, an absorbance meter, and a fluorescence reader). In addition, the kit preferably includes, as the standard samples, the protein molecules or their fragments composing the biomarker set.

[Examples]

The preceding investigation identified 22 proteins that were found to be related with schizophrenia (Patent Literature 1). With the aim of specifying the combination of biomarkers, which allow highly accurate discrimination, the following study was carried out.

1. Expression analysis by Western blot test

(1) Method

For the subjects composed of thirty patients with schizophrenia (15 men: $43.7 \pm 12.6$ years of age, 15 women: $43.1 \pm 8.9$ years of age) and thirty healthy subjects (15 men: $44.4 \pm 12.9$ years of age, 15 women: $43.2 \pm 9.6$ years of age), the amount of expression of 22 identified proteins was reconfirmed by the Western blot test method.

(2) Result

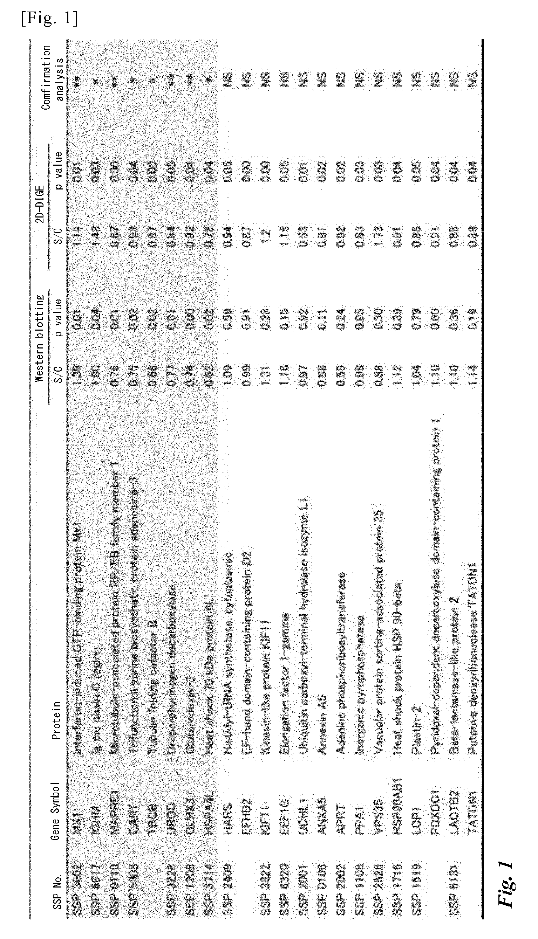

Of the 22 proteins, eight proteins showed a significant difference in the expression amount (Student's t-test, $p < 0.05$). Two proteins (MX1 and IGHM) showed increased expression, and six proteins (MAPRE1, TBCB, GLRX3, UROD, HSPA4L, and GART) showed decreased expression in the patient group with schizophrenia (Fig. 1).

2. Making of disease discriminant model using statistical method

The difference in the average of the amount of protein expression in the patient group with schizophrenia and the healthy subject group was compared using statistical hypothesis testing. As a result of this, eight proteins showed a significant difference (Student's t-test, $p < 0.05$) (same as the above-described result). Of the eight proteins, the protein useful for the discrimination of the patient group with schizophrenia and the healthy subject group were identified, and the statistical model that can highly accurately discriminate the disease (logistic model) was made. The logistic model is a statistical model for discriminating the binary response represented by the following formula (formula (1)) (herein, the patient with schizophrenia and healthy subject).

[Formula 2]

$$p = \frac{\exp(\beta_0 + \beta_1 \text{ Protein 1} + \cdots + \beta_k \text{ Protein k})}{1 + \exp(\beta_0 + \beta_1 \text{ Protein 1} + \cdots + \beta_k \text{ Protein k})} \qquad (1)$$

Wherein, p represents the probability of schizophrenia, and protein 1 to protein k represent the expression amount of the proteins used in discrimination. $\beta_0$ is the intercept and $\beta_1$ to $\beta_k$ are the parameters expressing the level of influence of the amounts of protein expression on the probability of schizophrenia and are presumed from the protein expression data. When $\beta$ is positive (negative), the higher the amount of protein expression, the higher (lower) the probability of schizophrenia. In order to make a highly accurate model for discriminating the disease, the optimum combination must be selected from all the 255 combinations of proteins (= $_8C_1 + _8C_2 + _8C_3 + _8C_4 + _8C_5 + _8C_6 + _8C_7 + _8C_8$). Using the statistical method below, two combinations of proteins (discriminant models) suitable for discrimination of the disease were identified.

<Discriminant model 1>

For the 255 protein combinations, the predicting errors based on the bias corrected area under the curve (AUC), and leave one out cross validation (LOOCV) were calculated. The closer the corrected AUC to 1.0, the smaller the predicting error based on LOOCV, the higher the discrimination accuracy of the combination. These indices are evaluation indices recommended in statistical textbooks and literatures. Fig. 2 shows the top 30 combinations of proteins having a high corrected AUC. The table in Fig. 2 indicates that the model using GART, UROD, MX1, GLRX3, MAPRE1, and TBCB (hereinafter referred to as discriminant model 1) achieved the highest discrimination accuracy (corrected AUC: 0.877, predicting error: 0.164). The logistic model of the discriminant model 1 is as follows (formula (2)).

[Formula 3]

$$p = \frac{\exp(11.54 - 4.64GART - 2.67UROD + 2.04MX1 - 3.14GLRX3 - 1.65MARPRE1 - 2.01TBCB)}{1 + \exp(11.54 - 4.64GART - 2.67UROD + 2.04MX1 - 3.14GLRX3 - 1.65MARPRE1 - 2.01TBCB)} \quad (2)$$

The formula (2) indicates that, the higher the MX1, the higher the probability of schizophrenia. For other proteins, the higher the value, the lower the probability of schizophrenia.

<Discriminant model 2>

Occam's razor is a statistical concept: "when an event is explained, the number of assumptions is preferably fewer". Specifically, when a dependent variant (presence or absence of schizophrenia) is explained using a statistical model based on the measurement data of a plurality of explanation variables (proteins), conformity to the "measurement data" is improved as the number of explaining variables included in the statistical model is increased to complicate the model. However, this statistical model excessively conforms to "the measurement data (past data)", and conformity to the new measured data decreases. More specifically, the statistical model overfits to the existing data. In order to avoid this problem, the number of explaining variables included in the statistical model is preferably fewer. Based on this concept, when the discrimination accuracy is on a similar level, the number of proteins used for the discrimination is preferably fewer. From the table in Fig. 2, the model using GART, UROD, MX1, and GLRX3 (hereinafter referred to as discriminant model 2) is suitable for the idea (corrected AUC: 0.860, predicting error: 0.178). The logistic model of the discriminant model 2 (formula (3)) is as follows.

[Formula 4]

$$p = \frac{\exp(6.44 - 3.82GART - 2.18UROD + 2.48MX1 - 2.84GLRX3)}{1 + \exp(6.44 - 3.82GART - 2.18UROD + 2.48MX1 - 2.84GLRX3)} \quad (3)$$

The positive and negative of the parameter are the same as those in the discriminant model 1, and the influences of the proteins on the probability of schizophrenia are the same as those in the discriminant models 1 and 2.

<Comparison between discriminant models 1 and 2>

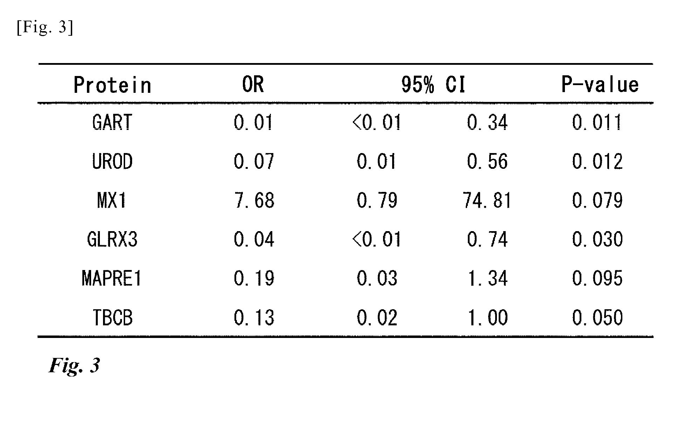

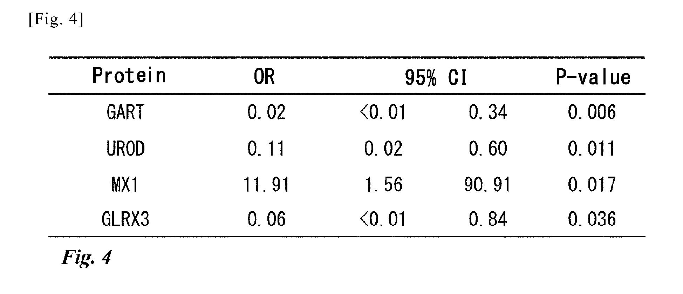

The odds ratios of the proteins contained in the discriminant models 1 and 2 are shown in Figs. 3 and 4, respectively. The influence of the protein contained in these models on the prevalence rate of schizophrenia is statistically significant ($p < 0.05$) or limitedly significant ($p < 0.10$), and is suggested to be a protein suitable for discrimination. In addition, the combination of proteins suitable for discrimination was selected using stepwise forward selection method, which is a statistical method. As a result of this, GART, UROD, MX1, and GLRX3 were selected, so that usefulness of the discriminant model 2 was supported by the different statistical method. Furthermore, the table in Fig. 2 shows that the number contained in the model is higher in the order of GART, UROD, MX1, GLRX3, MAPRE1, TBCB, IGHM, and HSPA4L.

The above results suggest the followings.

- The accuracy of discrimination of schizophrenia by the discriminant models 1 and 2 are almost equal statistically.
- GART, UROD, MX1, and GLRX3 contained in almost all the models in Fig. 2 are particularly important for the discrimination of schizophrenia.
- MARPRE1 and TBCB contained in the discriminant model 1 are also highly useful for discrimination.

<Determination of schizophrenia using discriminant models>

When determining whether the patient has schizophrenia or not using the discriminant model 1 or 2, the prevalence rate of schizophrenia (p in the formula (2) or (3)) is calculated. For example, when the discriminant model 1 is used, GART, UROD, MX1, GLRX3, MAPRE1, and TBCB in the patient are measured, and the values are assigned to the formula (2), thereby determining the prevalence rate of schizophrenia.

3. Validation of effectiveness of biomarker

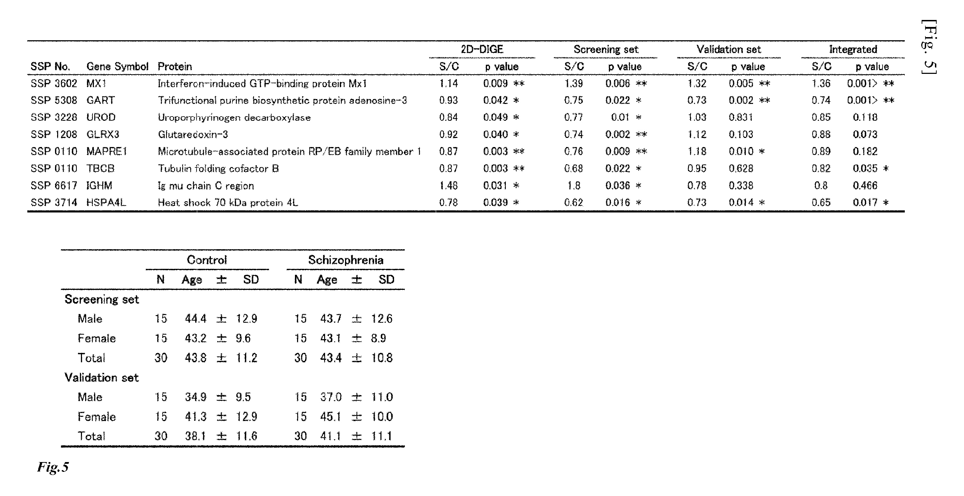

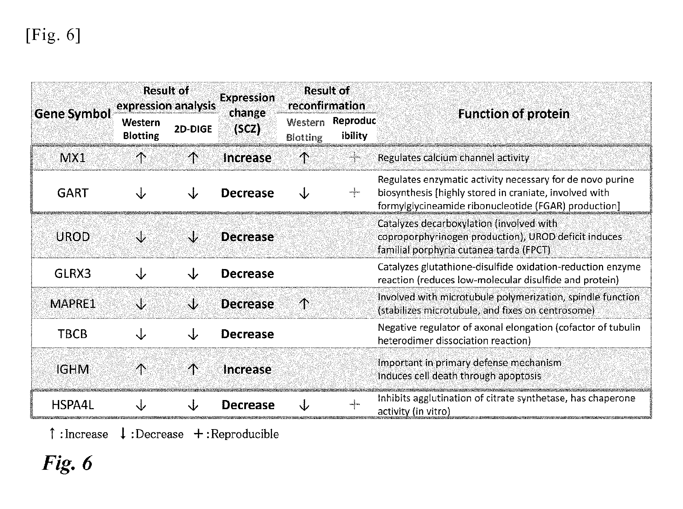

Effectiveness of the selected eight biomarkers was validated using other clinical specimens. The results of validation by Western blot test are shown in Figs. 5 and 6. For MX1, GART, and HSPA4L, the screening data (the data of 30 specimens used in the above-described 1.) and the validation data (the data of other 30 specimens) changed in the same direction (statistically significant), and showed reproducibility.

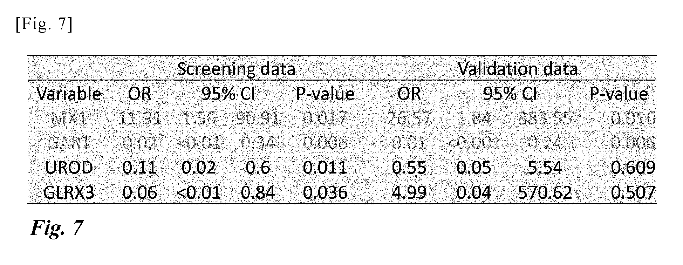

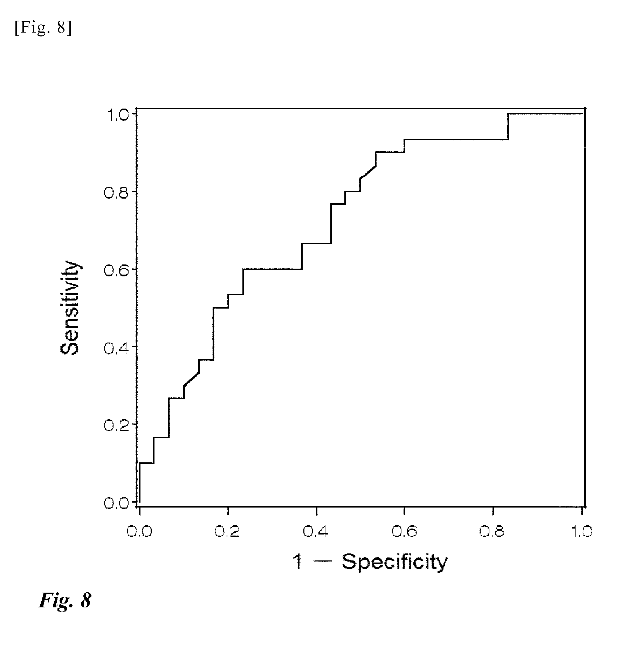

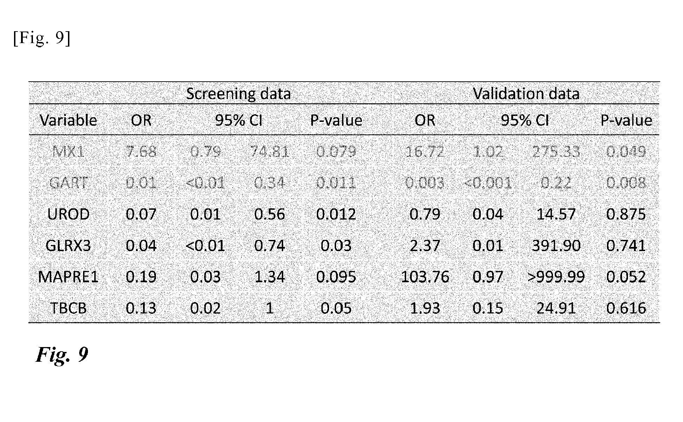

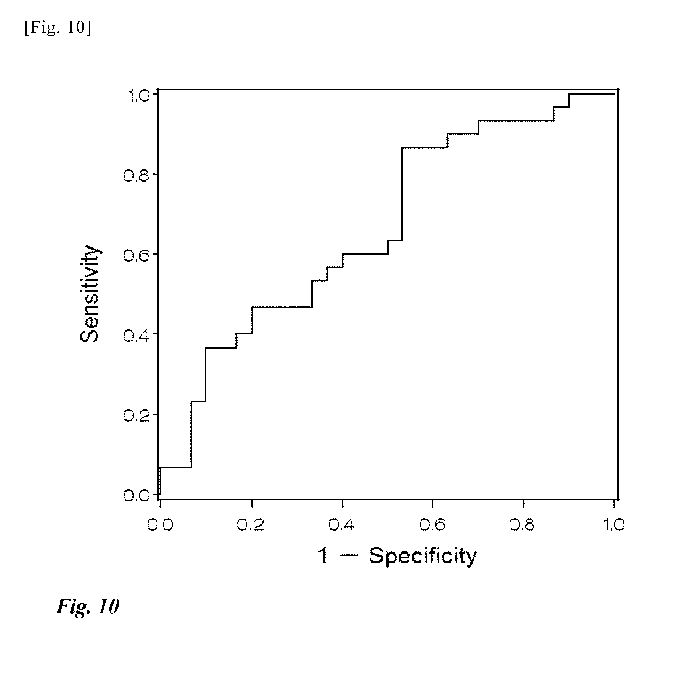

Effectiveness of the four biomarkers (MX1, GART, UROD, and GLRX3) was validated using the validation data set; significance was reproduced for MX1 and GART (Figs. 7 and 8). In addition, also when effectiveness of the six biomarkers (MX1, GART, UROD, GLRX3, MAPRE1, and TBCB) was validated using the validation data set, significance was reproduced for MX1 and GART (Figs. 9 and 10). The four-variable model showed a higher predicting accuracy.

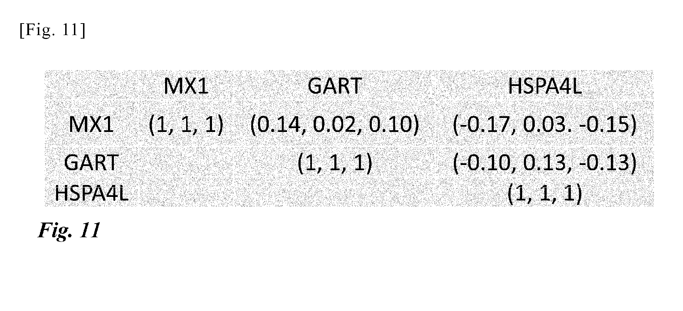

In consideration that MX1, GART, and HSPA4L showed reproducibility, the correlation between these three molecules was studied by multivariate analysis. The result is shown in Fig. 11. The values in parentheses are, from left to right, correlation coefficient in screening data, correlation coefficient in validation data, and correlation coefficient in the integration of both data. There is no correlation between the three molecules. The cause that HSPA4L showed no significance in the multivariate analysis is not likely due to the influence of multi-collinearity.

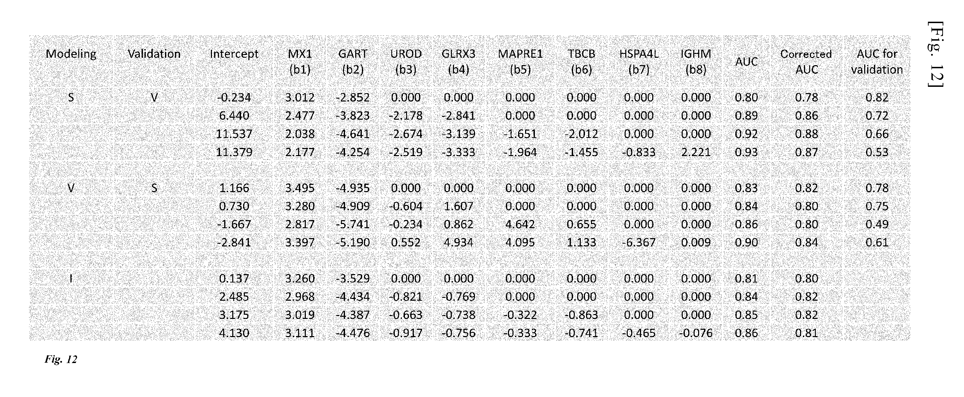

On the other hand, a plurality of predicting models including different types and numbers of biomarkers were constructed using eight biomarkers (MX1, GART, UROD, GLRX3, MAPRE1, TBCB, HSPA4L, IGHM), with focusing attention on MX1 and GART which reproduced significance, and further validation was carried out. The validation results are shown in Fig. 12. Except for some predicting models, high reproducibility is shown. From the viewpoints of predicting accuracy and reproducibility, the two variable models, MX1 and GART are the most advantageous. The predicting models are represented in the same manner as the above-described formula (1). For example, the discriminant model in the top row in Fig. 12 is represented by: $p = \exp(-0.234 + 3.012\text{MX1} - 2.852\text{GART}) / \{1 + \exp(-0.234 + 3.012\text{MX1} - 2.852\text{GART})$.

The above results suggest the followings.

- MX1 and GART have good reproducibility, and thus are advantageous biomarkers.
- HSPA4L also has relatively good reproducibility, and is highly useful.
- Discrimination using MX1 and GART gives the most accurate result.

[INDUSTRIAL APPLICABILITY]

The examination method of the present invention allows highly accurate discrimination of schizophrenia. The examination method of the present invention is useful as a means for determining whether schizophrenia is developed or not. It is also useful as a means for grasping the future development possibility. Early detection and treatment using the examination method of the present invention are considered effective for the prevention of increase in obstinacy and seriousness (progress of disease), and recurrence of schizophrenia.

The present invention will not be limited to the description of the embodiments and examples of the present invention. Various modifications readily made by those skilled in the art are also included in the present invention, without departing from the scope of claims. The entire contents of the articles, unexamined patent publications, patent applications, and the like specified herein are hereby incorporated herein by reference.

**Claims**

1. A schizophrenia marker set comprising a combination of two or more protein molecules selected from the group consisting of trifunctional purine biosynthetic protein adenosine-3, uroporphyrinogen decarboxylase, interferon-induced GTP-binding protein Mx1, glutaredoxin-3, microtubule-associated protein RP/EB family member 1, tubulin folding cofactor B, immunoglobulin mu chain C region, and heat shock 70 kDa protein 4L.

2. The schizophrenia marker set of claim 1, which comprises at least trifunctional purine biosynthetic protein adenosine-3, uroporphyrinogen decarboxylase, interferon-induced GTP-binding protein Mx1, and glutaredoxin-3.

3. The schizophrenia marker set of claim 2, which further comprises microtubule-associated protein RP/EB family member 1 and/or tubulin folding cofactor B.

4. The schizophrenia marker set of claim 1, which is a combination of trifunctional purine biosynthetic protein adenosine-3, uroporphyrinogen decarboxylase, interferon-induced GTP-binding protein Mx1, glutaredoxin-3, microtubule-associated protein RP/EB family member 1, and tubulin folding cofactor B.

5. The schizophrenia marker set of claim 1, which is a combination of trifunctional purine biosynthetic protein adenosine-3, uroporphyrinogen decarboxylase, interferon-induced GTP-binding protein Mx1, and glutaredoxin-3.

6. The schizophrenia marker set of claim 1, which comprises at least trifunctional purine biosynthetic protein adenosine-3 and interferon-induced GTP-binding protein Mx1.

7. The schizophrenia marker set of claim 1, which is a combination of trifunctional purine biosynthetic protein adenosine-3 and interferon-induced GTP-binding protein Mx1.

8. The schizophrenia marker set of claim 1, which comprises one or more protein molecules selected from the group consisting of trifunctional purine biosynthetic protein adenosine-3, interferon-induced GTP-binding protein Mx1, and heat shock 70 kDa protein 4L.

9. An examination method for schizophrenia using the level of the schizophrenia marker set of any one of claims 1 to 8 in a specimen as an indication.

10. The examination method for schizophrenia of claim 9, comprising the following steps (1) to (3):

    (1) a step of preparing a specimen derived from the subject;
    (2) a step of detecting the protein molecules composing the marker set in the specimen; and
    (3) a step of determining the present or future development possibility of schizophrenia based on the detection result.

11. The examination method for schizophrenia of claim 10, wherein a lower detected value shows a higher development possibility of schizophrenia for trifunctional purine biosynthetic protein adenosine-3, uroporphyrinogen decarboxylase, glutaredoxin-3, microtubule-associated protein RP/EB family member 1, tubulin folding cofactor B, and heat shock 70 kDa protein 4L, and a higher detected value shows a higher development possibility of schizophrenia for interferon-induced GTP-binding protein Mx1 and immunoglobulin mu chain C region.

12. The examination method for schizophrenia of claim 10 or 11, wherein the determination of step (3) is carried out using a logistic model.

13. The examination method for schizophrenia of any one of claims 9 to 12, wherein the specimen is blood, blood plasma, blood serum, oral mucosa, nasal mucosa, skin, blood cells, or lymphoblastoid cells prepared by immortalizing the blood lymphocytes collected from the subject.

14. A kit for examining schizophrenia comprising a reagent for detecting each of the protein molecules composing the schizophrenia marker set of claim 1, and an instruction manual.

15. The kit for examining schizophrenia of claim 14, wherein each of the reagents is an antibody to the target protein molecule.

EP 2 908 134 A1

[Fig. 1]

| SSP No. | Gene Symbol | Protein | Western blotting | | 2D-DIGE | | Comfirmation analysis |
|---|---|---|---|---|---|---|---|
| | | | S/C | p value | S/C | p value | |
| SSP 3602 | MX1 | Interferon-induced GTP-binding protein Mx1 | 1.39 | 0.01 | 1.14 | 0.01 | ** |
| SSP 6617 | IGHM | Ig mu chain C region | 1.80 | 0.04 | 1.48 | 0.03 | * |
| SSP 0110 | MAPRE1 | Microtubule-associated protein RP/EB family member 1 | 0.76 | 0.01 | 0.87 | 0.00 | ** |
| SSP 5308 | GART | Trifunctional purine biosynthetic protein adenosine-3 | 0.75 | 0.02 | 0.93 | 0.04 | * |
| | TBCB | Tubulin folding cofactor B | 0.68 | 0.02 | 0.87 | 0.00 | * |
| SSP 3228 | UROD | Uroporphyrinogen decarboxylase | 0.77 | 0.01 | 0.84 | 0.05 | ** |
| SSP 1208 | GLRX3 | Glutaredoxin-3 | 0.74 | 0.00 | 0.82 | 0.04 | ** |
| SSP 3714 | HSPA4L | Heat shock 70 kDa protein 4L | 0.62 | 0.02 | 0.78 | 0.04 | * |
| SSP 2409 | HARS | Histidyl-tRNA synthetase, cytoplasmic | 1.09 | 0.59 | 0.94 | 0.05 | NS |
| | EFHD2 | EF-hand domain-containing protein D2 | 0.99 | 0.91 | 0.87 | 0.00 | NS |
| SSP 3822 | KIF11 | Kinesin-like protein KIF11 | 1.31 | 0.28 | 1.2 | 0.00 | NS |
| SSP 6320 | EEF1G | Elongation factor 1-gamma | 1.16 | 0.15 | 1.18 | 0.05 | NS |
| SSP 2001 | UCHL1 | Ubiquitin carboxyl-terminal hydrolase isozyme L1 | 0.97 | 0.92 | 0.53 | 0.01 | NS |
| SSP 0106 | ANXA5 | Annexin A5 | 0.88 | 0.11 | 0.91 | 0.02 | NS |
| SSP 2002 | APRT | Adenine phosphoribosyltransferase | 0.59 | 0.24 | 0.92 | 0.02 | NS |
| SSP 1108 | PPA1 | Inorganic pyrophosphatase | 0.98 | 0.85 | 0.83 | 0.03 | NS |
| SSP 2628 | VPS35 | Vacuolar protein sorting-associated protein 35 | 0.88 | 0.30 | 1.73 | 0.03 | NS |
| SSP 1716 | HSP90AB1 | Heat shock protein HSP 90-beta | 1.12 | 0.39 | 0.91 | 0.04 | NS |
| SSP 1519 | LCP1 | Plastin-2 | 1.04 | 0.79 | 0.86 | 0.05 | NS |
| | PDXDC1 | Pyridoxal-dependent decarboxylase domain-containing protein 1 | 1.10 | 0.60 | 0.91 | 0.04 | NS |
| SSP 6131 | LACTB2 | Beta-lactamase-like protein 2 | 1.10 | 0.36 | 0.88 | 0.04 | NS |
| | TATDN1 | Putative deoxyribonuclease TATDN1 | 1.14 | 0.19 | 0.88 | 0.04 | NS |

*Fig. 1*

[Fig. 2]

| Number of proteins | Corrected AUC | Prediction error | Proteins included in Model | | | | | | | |
|---|---|---|---|---|---|---|---|---|---|---|
| 6 (Candidate Model 1) | 0.877 | 0.164 | GART | UROD | MX1 | GLRX3 | MAPRE1 | TBCB | | |
| 7 | 0.872 | 0.181 | GART | UROD | MX1 | GLRX3 | MAPRE1 | TBCB | IGHM | |
| 7 | 0.871 | 0.171 | GART | UROD | MX1 | GLRX3 | MAPRE1 | TBCB | | HSPL4L |
| 5 | 0.871 | 0.169 | GART | UROD | | GLRX3 | MAPRE1 | TBCB | | |
| 8 | 0.870 | 0.182 | GART | UROD | MX1 | GLRX3 | MAPRE1 | TBCB | IGHM | HSPL4L |
| 5 | 0.868 | 0.167 | GART | UROD | MX1 | | MAPRE1 | TBCB | | |
| 6 | 0.866 | 0.175 | GART | UROD | MX1 | GLRX3 | | TBCB | IGHM | |
| 6 | 0.865 | 0.174 | GART | UROD | MX1 | GLRX3 | MAPRE1 | | | HSPL4L |
| 6 | 0.863 | 0.174 | GART | UROD | MX1 | | MAPRE1 | TBCB | | HSPL4L |
| 6 | 0.863 | 0.173 | GART | UROD | | GLRX3 | MAPRE1 | TBCB | | HSPL4L |
| 7 | 0.863 | 0.180 | GART | UROD | MX1 | GLRX3 | MAPRE1 | | IGHM | HSPL4L |
| 6 | 0.862 | 0.178 | GART | | MX1 | GLRX3 | MAPRE1 | TBCB | IGHM | HSPL4L |
| 5 | 0.862 | 0.172 | GART | UROD | MX1 | GLRX3 | | TBCB | | HSPL4L |
| 6 | 0.860 | 0.177 | GART | UROD | | GLRX3 | MAPRE1 | TBCB | | |
| 4 (Candidate Model 2) | 0.860 | 0.178 | GART | UROD | MX1 | GLRX3 | | | | |
| 7 | 0.859 | 0.188 | GART | UROD | MX1 | GLRX3 | | TBCB | IGHM | HSPL4L |
| 6 | 0.857 | 0.177 | GART | UROD | MX1 | | MAPRE1 | | IGHM | |
| 7 | 0.856 | 0.185 | GART | UROD | MX1 | | MAPRE1 | TBCB | IGHM | |
| 5 | 0.856 | 0.186 | GART | UROD | MX1 | GLRX3 | MAPRE1 | | IGHM | |
| 7 | 0.855 | 0.180 | GART | UROD | | GLRX3 | MAPRE1 | TBCB | IGHM | HSPL4L |
| 6 | 0.855 | 0.183 | GART | UROD | MX1 | GLRX3 | | TBCB | | HSPL4L |
| 6 | 0.854 | 0.176 | GART | UROD | MX1 | | MAPRE1 | TBCB | IGHM | |
| 5 | 0.853 | 0.179 | GART | UROD | MX1 | GLRX3 | | | | HSPL4L |
| 6 | 0.853 | 0.184 | GART | | MX1 | GLRX3 | MAPRE1 | | IGHM | HSPL4L |
| 6 | 0.853 | 0.182 | GART | UROD | MX1 | GLRX3 | | | IGHM | HSPL4L |
| 5 | 0.852 | 0.180 | GART | UROD | MX1 | GLRX3 | | | IGHM | |
| 7 | 0.850 | 0.192 | GART | | MX1 | GLRX3 | MAPRE1 | TBCB | IGHM | HSPL4L |
| 6 | 0.848 | 0.180 | GART | UROD | MX1 | GLRX3 | MAPRE1 | | IGHM | |
| 5 | 0.826 | 0.189 | GART | UROD | MX1 | | | TBCB | IGHM | |
| 6 | 0.824 | 0.200 | GART | UROD | MX1 | | | TBCB | IGHM | HSPL4L |

*Fig. 2*

[Fig. 3]

| Protein | OR | 95% CI | | P-value |
|---------|-----|--------|--------|---------|
| GART | 0.01 | <0.01 | 0.34 | 0.011 |
| UROD | 0.07 | 0.01 | 0.56 | 0.012 |
| MX1 | 7.68 | 0.79 | 74.81 | 0.079 |
| GLRX3 | 0.04 | <0.01 | 0.74 | 0.030 |
| MAPRE1 | 0.19 | 0.03 | 1.34 | 0.095 |
| TBCB | 0.13 | 0.02 | 1.00 | 0.050 |

*Fig. 3*

[Fig. 4]

| Protein | OR | 95% CI | | P-value |
|---------|-----|--------|------|---------|
| GART | 0.02 | <0.01 | 0.34 | 0.006 |
| UROD | 0.11 | 0.02 | 0.60 | 0.011 |
| MX1 | 11.91 | 1.56 | 90.91 | 0.017 |
| GLRX3 | 0.06 | <0.01 | 0.84 | 0.036 |

*Fig. 4*

[Fig. 5]

EP 2 908 134 A1

| SSP No. | Gene Symbol | Protein | 2D-DIGE | | Screening set | | Validation set | | Integrated | |
|---|---|---|---|---|---|---|---|---|---|---|
| | | | S/C | p value | S/C | p value | S/C | p value | S/C | p value |
| SSP 3602 | MX1 | Interferon-induced GTP-binding protein Mx1 | 1.14 | 0.009 ** | 1.39 | 0.006 ** | 1.32 | 0.005 ** | 1.36 | 0.001> ** |
| SSP 5308 | GART | Trifunctional purine biosynthetic protein adenosine-3 | 0.93 | 0.042 * | 0.75 | 0.022 * | 0.73 | 0.002 ** | 0.74 | 0.001> ** |
| SSP 3228 | UROD | Uroporphyrinogen decarboxylase | 0.84 | 0.049 * | 0.77 | 0.01 * | 1.03 | 0.831 | 0.85 | 0.118 |
| SSP 1208 | GLRX3 | Glutaredoxin-3 | 0.92 | 0.040 * | 0.74 | 0.002 ** | 1.12 | 0.103 | 0.88 | 0.073 |
| SSP 0110 | MAPRE1 | Microtubule-associated protein RP/EB family member 1 | 0.87 | 0.003 ** | 0.76 | 0.009 ** | 1.18 | 0.010 * | 0.89 | 0.182 |
| SSP 0110 | TBCB | Tubulin folding cofactor B | 0.87 | 0.003 ** | 0.68 | 0.022 * | 0.95 | 0.628 | 0.82 | 0.035 * |
| SSP 6617 | IGHM | Ig mu chain C region | 1.48 | 0.031 * | 1.8 | 0.036 * | 0.78 | 0.338 | 0.8 | 0.466 |
| SSP 3714 | HSPA4L | Heat shock 70 kDa protein 4L | 0.78 | 0.039 * | 0.62 | 0.016 * | 0.73 | 0.014 * | 0.65 | 0.017 * |

| | Control | | | | Schizophrenia | | | |
|---|---|---|---|---|---|---|---|---|
| | N | Age | ± | SD | N | Age | ± | SD |
| **Screening set** | | | | | | | | |
| Male | 15 | 44.4 | ± | 12.9 | 15 | 43.7 | ± | 12.6 |
| Female | 15 | 43.2 | ± | 9.6 | 15 | 43.1 | ± | 8.9 |
| Total | 30 | 43.8 | ± | 11.2 | 30 | 43.4 | ± | 10.8 |
| **Validation set** | | | | | | | | |
| Male | 15 | 34.9 | ± | 9.5 | 15 | 37.0 | ± | 11.0 |
| Female | 15 | 41.3 | ± | 12.9 | 15 | 45.1 | ± | 10.0 |
| Total | 30 | 38.1 | ± | 11.6 | 30 | 41.1 | ± | 11.1 |

*Fig.5*

[Fig. 6]

| Gene Symbol | Result of expression analysis | | Expression change (SCZ) | Result of reconfirmation | | Function of protein |
|---|---|---|---|---|---|---|
| | Western Blotting | 2D-DIGE | | Western Blotting | Reproducibility | |
| MX1 | ↑ | ↑ | Increase | ↑ | + | Regulates calcium channel activity |
| GART | ↓ | ↓ | Decrease | ↓ | + | Regulates enzymatic activity necessary for de novo purine biosynthesis [highly stored in craniate, involved with formylglycineamide ribonucleotide (FGAR) production] |
| UROD | ↓ | ↓ | Decrease | | | Catalyzes decarboxylation (involved with coproporphyrinogen production), UROD deficit induces familial porphyria cutanea tarda (FPCT) |
| GLRX3 | ↓ | ↓ | Decrease | | | Catalyzes glutathione-disulfide oxidation-reduction enzyme reaction (reduces low-molecular disulfide and protein) |
| MAPRE1 | ↓ | ↓ | Decrease | ↑ | | Involved with microtubule polymerization, spindle function (stabilizes microtubule, and fixes on centrosome) |
| TBCB | ↓ | ↓ | Decrease | | | Negative regulator of axonal elongation (cofactor of tubulin heterodimer dissociation reaction) |
| IGHM | ↑ | ↑ | Increase | | | Important in primary defense mechanism Induces cell death through apoptosis |
| HSPA4L | ↓ | ↓ | Decrease | ↓ | + | Inhibits agglutination of citrate synthetase, has chaperone activity (in vitro) |

↑ :Increase　↓ :Decrease　+ :Reproducible

*Fig. 6*

[Fig. 7]

| | Screening data | | | | Validation data | | |
|---|---|---|---|---|---|---|---|
| Variable | OR | 95% CI | | P-value | OR | 95% CI | | P-value |
| MX1 | 11.91 | 1.56 | 90.91 | 0.017 | 26.57 | 1.84 | 383.55 | 0.016 |
| GART | 0.02 | <0.01 | 0.34 | 0.006 | 0.01 | <0.001 | 0.24 | 0.006 |
| UROD | 0.11 | 0.02 | 0.6 | 0.011 | 0.55 | 0.05 | 5.54 | 0.609 |
| GLRX3 | 0.06 | <0.01 | 0.84 | 0.036 | 4.99 | 0.04 | 570.62 | 0.507 |

*Fig. 7*

[Fig. 8]

*Fig. 8*

[Fig. 9]

| | Screening data | | | | Validation data | | | |
|---|---|---|---|---|---|---|---|---|
| Variable | OR | 95% CI | | P-value | OR | 95% CI | | P-value |
| MX1 | 7.68 | 0.79 | 74.81 | 0.079 | 16.72 | 1.02 | 275.33 | 0.049 |
| GART | 0.01 | <0.01 | 0.34 | 0.011 | 0.003 | <0.001 | 0.22 | 0.008 |
| UROD | 0.07 | 0.01 | 0.56 | 0.012 | 0.79 | 0.04 | 14.57 | 0.875 |
| GLRX3 | 0.04 | <0.01 | 0.74 | 0.03 | 2.37 | 0.01 | 391.90 | 0.741 |
| MAPRE1 | 0.19 | 0.03 | 1.34 | 0.095 | 103.76 | 0.97 | >999.99 | 0.052 |
| TBCB | 0.13 | 0.02 | 1 | 0.05 | 1.93 | 0.15 | 24.91 | 0.616 |

*Fig. 9*

[Fig. 10]

*Fig. 10*

[Fig. 11]

|        | MX1       | GART                | HSPA4L               |
|--------|-----------|---------------------|----------------------|
| MX1    | (1, 1, 1) | (0.14, 0.02, 0.10)  | (-0.17, 0.03. -0.15) |
| GART   |           | (1, 1, 1)           | (-0.10, 0.13, -0.13) |
| HSPA4L |           |                     | (1, 1, 1)            |

*Fig. 11*

[Fig. 12]

| Modeling | Validation | Intercept | MX1 (b1) | GART (b2) | UROD (b3) | GLRX3 (b4) | MAPRE1 (b5) | TBCB (b6) | HSPA4L (b7) | IGHM (b8) | AUC | Corrected AUC | AUC for validation |
|---|---|---|---|---|---|---|---|---|---|---|---|---|---|
| S | V | -0.234 | 3.012 | -2.852 | 0.000 | 0.000 | 0.000 | 0.000 | 0.000 | 0.000 | 0.80 | 0.78 | 0.82 |
|   |   | 6.440 | 2.477 | -3.823 | -2.178 | -2.841 | 0.000 | 0.000 | 0.000 | 0.000 | 0.89 | 0.86 | 0.72 |
|   |   | 11.537 | 2.038 | -4.641 | -2.674 | -3.139 | -1.651 | -2.012 | 0.000 | 0.000 | 0.92 | 0.88 | 0.66 |
|   |   | 11.379 | 2.177 | -4.254 | -2.519 | -3.333 | -1.964 | -1.455 | -0.833 | 2.221 | 0.93 | 0.87 | 0.53 |
| V | S | 1.166 | 3.495 | -4.935 | 0.000 | 0.000 | 0.000 | 0.000 | 0.000 | 0.000 | 0.83 | 0.82 | 0.78 |
|   |   | 0.730 | 3.280 | -4.909 | -0.604 | 1.607 | 0.000 | 0.000 | 0.000 | 0.000 | 0.84 | 0.80 | 0.75 |
|   |   | -1.667 | 2.817 | -5.741 | -0.234 | 0.862 | 4.642 | 0.655 | 0.000 | 0.000 | 0.86 | 0.80 | 0.49 |
|   |   | -2.841 | 3.397 | -5.190 | 0.552 | 4.934 | 4.095 | 1.133 | -6.367 | 0.009 | 0.90 | 0.84 | 0.61 |
| I |   | 0.137 | 3.260 | -3.529 | 0.000 | 0.000 | 0.000 | 0.000 | 0.000 | 0.000 | 0.81 | 0.80 |  |
|   |   | 2.485 | 2.968 | -4.434 | -0.821 | -0.769 | 0.000 | 0.000 | 0.000 | 0.000 | 0.84 | 0.82 |  |
|   |   | 3.175 | 3.019 | -4.387 | -0.663 | -0.738 | -0.322 | -0.863 | 0.000 | 0.000 | 0.85 | 0.82 |  |
|   |   | 4.130 | 3.111 | -4.476 | -0.917 | -0.756 | -0.333 | -0.741 | -0.465 | -0.076 | 0.86 | 0.81 |  |

Fig. 12

# EP 2 908 134 A1

## INTERNATIONAL SEARCH REPORT

International application No.

PCT/JP2013/076918

### A. CLASSIFICATION OF SUBJECT MATTER
*G01N33/53*(2006.01)i

According to International Patent Classification (IPC) or to both national classification and IPC

### B. FIELDS SEARCHED

Minimum documentation searched (classification system followed by classification symbols)
G01N33/53

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2012-13415 A  (Nagoya University), 19 January 2012 (19.01.2012), claims 1, 3; paragraphs [0030], [0031], [0006]; claims 7, 11; paragraph [0043] (Family: none) | 1-8,14,15 |
| A | JP 2009-510395 A  (The Regents of the University of California), 12 March 2009 (12.03.2009), paragraph [0071] & US 2007/0117123 A1    & EP 1934373 A | 1-8,14,15 |

☐ Further documents are listed in the continuation of Box C.   ☐ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search <br> 16 October, 2013 (16.10.13) | Date of mailing of the international search report <br> 29 October, 2013 (29.10.13) |
|---|---|
| Name and mailing address of the ISA/ <br> Japanese Patent Office | Authorized officer |
| Facsimile No. | Telephone No. |

Form PCT/ISA/210 (second sheet) (July 2009)

**44**

## INTERNATIONAL SEARCH REPORT

International application No.

PCT/JP2013/076918

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 9-13
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2013/076918

Continuation of Box No.II-1 of continuation of first sheet(2)

Claims 9 to 13 pertain to "examination methods of integration disorder syndrome" that correspond to diagnostic methods to be practiced on the human body and thus relate to a subject matter which this International Searching Authority is not required, under the provisions of Article 17(2)(a)(i) of the PCT and Rule 39.1(iv) of the Regulations under the PCT, to search.

Form PCT/ISA/210 (extra sheet) (July 2009)

**EP 2 908 134 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012228417 A

- JP 2012013415 A

**Non-patent literature cited in the description**

- **ARCOS-BURGOS M et al.** *Am J Hum Genet,* 2005, vol. 77 (6), 937-44
- **LEWIS CM et al.** *Am J Hum Genet,* 2003, vol. 73, 34-48

- **BADNER JA. ; GERSHON ES.** *Mol Psychiatry,* 2002, vol. 7, 405-11
- **TAKAHASHI et al.** *Psychiatry Res,* 2007, vol. 154, 209-219

[SEQUENCE LIST]

SEQUENCE LISTING

<110> NATIONAL UNIVERSITY CORPORATION NAGOYA UNIVERSITY

<120> SET OF BIOMARKER FOR SCHIZOPHRENIA AND USE THEREOF

<130> NU12008P

<150> JP P2012-228417
<151> 2012-10-15

<160> 8

<170> PatentIn version 3.5

<210> 1
<211> 1010
<212> PRT
<213> Homo sapiens

<400> 1

Met Ala Ala Arg Val Leu Ile Ile Gly Ser Gly Gly Arg Glu His Thr
1               5                   10                  15


Leu Ala Trp Lys Leu Ala Gln Ser His His Val Lys Gln Val Leu Val
                20                  25                  30


Ala Pro Gly Asn Ala Gly Thr Ala Cys Ser Glu Lys Ile Ser Asn Thr
            35                  40                  45


Ala Ile Ser Ile Ser Asp His Thr Ala Leu Ala Gln Phe Cys Lys Glu
        50                  55                  60


Lys Lys Ile Glu Phe Val Val Val Gly Pro Glu Ala Pro Leu Ala Ala
65                  70                  75                  80


Gly Ile Val Gly Asn Leu Arg Ser Ala Gly Val Gln Cys Phe Gly Pro
                85                  90                  95


Thr Ala Glu Ala Ala Gln Leu Glu Ser Ser Lys Arg Phe Ala Lys Glu
            100                 105                 110


Phe Met Asp Arg His Gly Ile Pro Thr Ala Gln Trp Lys Ala Phe Thr
            115                 120                 125


Lys Pro Glu Glu Ala Cys Ser Phe Ile Leu Ser Ala Asp Phe Pro Ala
        130                 135                 140


Leu Val Val Lys Ala Ser Gly Leu Ala Ala Gly Lys Gly Val Ile Val
145                 150                 155                 160


Ala Lys Ser Lys Glu Glu Ala Cys Lys Ala Val Gln Glu Ile Met Gln

```
                   165                      170                         175

    Glu Lys Ala Phe Gly Ala Ala Gly Glu Thr Ile Val Ile Glu Glu Leu
                180                 185                 190

    Leu Asp Gly Glu Glu Val Ser Cys Leu Cys Phe Thr Asp Gly Lys Thr
                195                 200                 205

    Val Ala Pro Met Pro Pro Ala Gln Asp His Lys Arg Leu Leu Glu Gly
            210                 215                 220

    Asp Gly Gly Pro Asn Thr Gly Gly Met Gly Ala Tyr Cys Pro Ala Pro
    225                 230                 235                 240

    Gln Val Ser Asn Asp Leu Leu Leu Lys Ile Lys Asp Thr Val Leu Gln
                245                 250                 255

    Arg Thr Val Asp Gly Met Gln Gln Glu Gly Thr Pro Tyr Thr Gly Ile
                260                 265                 270

    Leu Tyr Ala Gly Ile Met Leu Thr Lys Asn Gly Pro Lys Val Leu Glu
                275                 280                 285

    Phe Asn Cys Arg Phe Gly Asp Pro Glu Cys Gln Val Ile Leu Pro Leu
            290                 295                 300

    Leu Lys Ser Asp Leu Tyr Glu Val Ile Gln Ser Thr Leu Asp Gly Leu
    305                 310                 315                 320

    Leu Cys Thr Ser Leu Pro Val Trp Leu Glu Asn His Thr Ala Leu Thr
                325                 330                 335

    Val Val Met Ala Ser Lys Gly Tyr Pro Gly Asp Tyr Thr Lys Gly Val
                340                 345                 350

    Glu Ile Thr Gly Phe Pro Glu Ala Gln Ala Leu Gly Leu Glu Val Phe
            355                 360                 365

    His Ala Gly Thr Ala Leu Lys Asn Gly Lys Val Val Thr His Gly Gly
            370                 375                 380

    Arg Val Leu Ala Val Thr Ala Ile Arg Glu Asn Leu Ile Ser Ala Leu
    385                 390                 395                 400

    Glu Glu Ala Lys Lys Gly Leu Ala Ala Ile Lys Phe Glu Gly Ala Ile
                405                 410                 415
```

```
Tyr Arg Lys Asp Val Gly Phe Arg Ala Ile Ala Phe Leu Gln Gln Pro
            420             425             430

Arg Ser Leu Thr Tyr Lys Glu Ser Gly Val Asp Ile Ala Ala Gly Asn
            435             440             445

Met Leu Val Lys Lys Ile Gln Pro Leu Ala Lys Ala Thr Ser Arg Ser
            450             455             460

Gly Cys Lys Val Asp Leu Gly Gly Phe Ala Gly Leu Phe Asp Leu Lys
465             470             475             480

Ala Ala Gly Phe Lys Asp Pro Leu Leu Ala Ser Gly Thr Asp Gly Val
                485             490             495

Gly Thr Lys Leu Lys Ile Ala Gln Leu Cys Asn Lys His Asp Thr Ile
            500             505             510

Gly Gln Asp Leu Val Ala Met Cys Val Asn Asp Ile Leu Ala Gln Gly
            515             520             525

Ala Glu Pro Leu Phe Phe Leu Asp Tyr Phe Ser Cys Gly Lys Leu Asp
            530             535             540

Leu Ser Val Thr Glu Ala Val Ala Gly Ile Ala Lys Ala Cys Gly
545             550             555             560

Lys Ala Gly Cys Ala Leu Leu Gly Gly Glu Thr Ala Glu Met Pro Asp
                565             570             575

Met Tyr Pro Pro Gly Glu Tyr Asp Leu Ala Gly Phe Ala Val Gly Ala
            580             585             590

Met Glu Arg Asp Gln Lys Leu Pro His Leu Glu Arg Ile Thr Glu Gly
            595             600             605

Asp Val Val Val Gly Ile Ala Ser Ser Gly Leu His Ser Asn Gly Phe
            610             615             620

Ser Leu Val Arg Lys Ile Val Ala Lys Ser Ser Leu Gln Tyr Ser Ser
625             630             635             640

Pro Ala Pro Asp Gly Cys Gly Asp Gln Thr Leu Gly Asp Leu Leu Leu
                645             650             655

Thr Pro Thr Arg Ile Tyr Ser His Ser Leu Leu Pro Val Leu Arg Ser
                660             665             670
```

Gly His Val Lys Ala Phe Ala His Ile Thr Gly Gly Gly Leu Leu Glu
675                     680                 685

Asn Ile Pro Arg Val Leu Pro Glu Lys Leu Gly Val Asp Leu Asp Ala
690                     695                 700

Gln Thr Trp Arg Ile Pro Arg Val Phe Ser Trp Leu Gln Gln Glu Gly
705                 710                 715                 720

His Leu Ser Glu Glu Glu Met Ala Arg Thr Phe Asn Cys Gly Val Gly
725                 730                 735

Ala Val Leu Val Val Ser Lys Glu Gln Thr Glu Gln Ile Leu Arg Asp
740                 745                 750

Ile Gln Gln His Lys Glu Glu Ala Trp Val Ile Gly Ser Val Val Ala
755                 760                 765

Arg Ala Glu Gly Ser Pro Arg Val Lys Val Lys Asn Leu Ile Glu Ser
770                 775                 780

Met Gln Ile Asn Gly Ser Val Leu Lys Asn Gly Ser Leu Thr Asn His
785                 790                 795                 800

Phe Ser Phe Glu Lys Lys Lys Ala Arg Val Ala Val Leu Ile Ser Gly
805                 810                 815

Thr Gly Ser Asn Leu Gln Ala Leu Ile Asp Ser Thr Arg Glu Pro Asn
820                 825                 830

Ser Ser Ala Gln Ile Asp Ile Val Ile Ser Asn Lys Ala Ala Val Ala
835                 840                 845

Gly Leu Asp Lys Ala Glu Arg Ala Gly Ile Pro Thr Arg Val Ile Asn
850                 855                 860

His Lys Leu Tyr Lys Asn Arg Val Glu Phe Asp Ser Ala Ile Asp Leu
865                 870                 875                 880

Val Leu Glu Glu Phe Ser Ile Asp Ile Val Cys Leu Ala Gly Phe Met
885                 890                 895

Arg Ile Leu Ser Gly Pro Phe Val Gln Lys Trp Asn Gly Lys Met Leu
900                 905                 910

Asn Ile His Pro Ser Leu Leu Pro Ser Phe Lys Gly Ser Asn Ala His
915                 920                 925

16

```
Glu Gln Ala Leu Glu Thr Gly Val Thr Val Thr Gly Cys Thr Val His
    930                 935             940

Phe Val Ala Glu Asp Val Asp Ala Gly Gln Ile Ile Leu Gln Glu Ala
945                 950             955                 960

Val Pro Val Lys Arg Gly Asp Thr Val Ala Thr Leu Ser Glu Arg Val
            965             970                 975

Lys Leu Ala Glu His Lys Ile Phe Pro Ala Ala Leu Gln Leu Val Ala
            980             985             990

Ser Gly Thr Val Gln Leu Gly Glu Asn Gly Lys Ile Cys Trp Val Lys
        995             1000            1005

Glu Glu
    1010


<210>  2
<211>  367
<212>  PRT
<213>  Homo sapiens

<400>  2

Met Glu Ala Asn Gly Leu Gly Pro Gln Gly Phe Pro Glu Leu Lys Asn
1               5               10                  15

Asp Thr Phe Leu Arg Ala Ala Trp Gly Glu Glu Thr Asp Tyr Thr Pro
            20              25              30

Val Trp Cys Met Arg Gln Ala Gly Arg Tyr Leu Pro Glu Phe Arg Glu
        35              40              45

Thr Arg Ala Ala Gln Asp Phe Phe Ser Thr Cys Arg Ser Pro Glu Ala
    50              55              60

Cys Cys Glu Leu Thr Leu Gln Pro Leu Arg Arg Phe Pro Leu Asp Ala
65              70              75                  80

Ala Ile Ile Phe Ser Asp Ile Leu Val Val Pro Gln Ala Leu Gly Met
            85              90                  95

Glu Val Thr Met Val Pro Gly Lys Gly Pro Ser Phe Pro Glu Pro Leu
            100             105             110

Arg Glu Glu Gln Asp Leu Glu Arg Leu Arg Asp Pro Glu Val Val Ala
        115             120             125
```

Ser Glu Leu Gly Tyr Val Phe Gln Ala Ile Thr Leu Thr Arg Gln Arg
130                 135                 140

Leu Ala Gly Arg Val Pro Leu Ile Gly Phe Ala Gly Ala Pro Trp Thr
145                 150                 155                 160

Leu Met Thr Tyr Met Val Glu Gly Gly Gly Ser Ser Thr Met Ala Gln
                165                 170                 175

Ala Lys Arg Trp Leu Tyr Gln Arg Pro Gln Ala Ser His Gln Leu Leu
            180                 185                 190

Arg Ile Leu Thr Asp Ala Leu Val Pro Tyr Leu Val Gly Gln Val Val
            195                 200                 205

Ala Gly Ala Gln Ala Leu Gln Leu Phe Glu Ser His Ala Gly His Leu
210                 215                 220

Gly Pro Gln Leu Phe Asn Lys Phe Ala Leu Pro Tyr Ile Arg Asp Val
225                 230                 235                 240

Ala Lys Gln Val Lys Ala Arg Leu Arg Glu Ala Gly Leu Ala Pro Val
            245                 250                 255

Pro Met Ile Ile Phe Ala Lys Asp Gly His Phe Ala Leu Glu Glu Leu
            260                 265                 270

Ala Gln Ala Gly Tyr Glu Val Val Gly Leu Asp Trp Thr Val Ala Pro
            275                 280                 285

Lys Lys Ala Arg Glu Cys Val Gly Lys Thr Val Thr Leu Gln Gly Asn
290                 295                 300

Leu Asp Pro Cys Ala Leu Tyr Ala Ser Glu Glu Glu Ile Gly Gln Leu
305                 310                 315                 320

Val Lys Gln Met Leu Asp Asp Phe Gly Pro His Arg Tyr Ile Ala Asn
                325                 330                 335

Leu Gly His Gly Leu Tyr Pro Asp Met Asp Pro Glu His Val Gly Ala
                340                 345                 350

Phe Val Asp Ala Val His Lys His Ser Arg Leu Leu Arg Gln Asn
                355                 360                 365

<210> 3

EP 2 908 134 A1

<211> 662
<212> PRT
<213> Homo sapiens

<400> 3

```
Met Val Val Ser Glu Val Asp Ile Ala Lys Ala Asp Pro Ala Ala Ala
1               5               10              15

Ser His Pro Leu Leu Leu Asn Gly Asp Ala Thr Val Ala Gln Lys Asn
            20              25              30

Pro Gly Ser Val Ala Glu Asn Asn Leu Cys Ser Gln Tyr Glu Glu Lys
            35              40              45

Val Arg Pro Cys Ile Asp Leu Ile Asp Ser Leu Arg Ala Leu Gly Val
        50              55              60

Glu Gln Asp Leu Ala Leu Pro Ala Ile Ala Val Ile Gly Asp Gln Ser
65              70              75              80

Ser Gly Lys Ser Ser Val Leu Glu Ala Leu Ser Gly Val Ala Leu Pro
                85              90              95

Arg Gly Ser Gly Ile Val Thr Arg Cys Pro Leu Val Leu Lys Leu Lys
            100             105             110

Lys Leu Val Asn Glu Asp Lys Trp Arg Gly Lys Val Ser Tyr Gln Asp
            115             120             125

Tyr Glu Ile Glu Ile Ser Asp Ala Ser Glu Val Glu Lys Glu Ile Asn
    130             135             140

Lys Ala Gln Asn Ala Ile Ala Gly Glu Gly Met Gly Ile Ser His Glu
145             150             155             160

Leu Ile Thr Leu Glu Ile Ser Ser Arg Asp Val Pro Asp Leu Thr Leu
                165             170             175

Ile Asp Leu Pro Gly Ile Thr Arg Val Ala Val Gly Asn Gln Pro Ala
            180             185             190

Asp Ile Gly Tyr Lys Ile Lys Thr Leu Ile Lys Lys Tyr Ile Gln Arg
            195             200             205

Gln Glu Thr Ile Ser Leu Val Val Val Pro Ser Asn Val Asp Ile Ala
    210             215             220

Thr Thr Glu Ala Leu Ser Met Ala Gln Glu Val Asp Pro Glu Gly Asp
```

19

```
                225                 230                 235                 240


Arg Thr Ile Gly Ile Leu Thr Lys Pro Asp Leu Val Asp Lys Gly Thr
            245             250                 255


Glu Asp Lys Val Val Asp Val Val Arg Asn Leu Val Phe His Leu Lys
            260             265                 270


Lys Gly Tyr Met Ile Val Lys Cys Arg Gly Gln Gln Glu Ile Gln Asp
            275             280                 285


Gln Leu Ser Leu Ser Glu Ala Leu Gln Arg Glu Lys Ile Phe Phe Glu
    290             295                 300


Asn His Pro Tyr Phe Arg Asp Leu Leu Glu Glu Gly Lys Ala Thr Val
305             310                 315                 320


Pro Cys Leu Ala Glu Lys Leu Thr Ser Glu Leu Ile Thr His Ile Cys
            325             330                 335


Lys Ser Leu Pro Leu Leu Glu Asn Gln Ile Lys Glu Thr His Gln Arg
            340             345                 350


Ile Thr Glu Glu Leu Gln Lys Tyr Gly Val Asp Ile Pro Glu Asp Glu
    355             360                 365


Asn Glu Lys Met Phe Phe Leu Ile Asp Lys Val Asn Ala Phe Asn Gln
    370             375                 380


Asp Ile Thr Ala Leu Met Gln Gly Glu Glu Thr Val Gly Glu Glu Asp
385             390                 395                 400


Ile Arg Leu Phe Thr Arg Leu Arg His Glu Phe His Lys Trp Ser Thr
            405             410                 415


Ile Ile Glu Asn Asn Phe Gln Glu Gly His Lys Ile Leu Ser Arg Lys
            420             425                 430


Ile Gln Lys Phe Glu Asn Gln Tyr Arg Gly Arg Glu Leu Pro Gly Phe
            435             440                 445


Val Asn Tyr Arg Thr Phe Glu Thr Ile Val Lys Gln Gln Ile Lys Ala
    450             455                 460


Leu Glu Glu Pro Ala Val Asp Met Leu His Thr Val Thr Asp Met Val
465             470                 475                 480
```

```
Arg Leu Ala Phe Thr Asp Val Ser Ile Lys Asn Phe Glu Glu Phe Phe
            485                 490                 495

Asn Leu His Arg Thr Ala Lys Ser Lys Ile Glu Asp Ile Arg Ala Glu
            500                 505                 510

Gln Glu Arg Glu Gly Glu Lys Leu Ile Arg Leu His Phe Gln Met Glu
            515                 520                 525

Gln Ile Val Tyr Cys Gln Asp Gln Val Tyr Arg Gly Ala Leu Gln Lys
        530                 535                 540

Val Arg Glu Lys Glu Leu Glu Glu Glu Lys Lys Lys Lys Ser Trp Asp
    545                 550                 555                 560

Phe Gly Ala Phe Gln Ser Ser Ser Ala Thr Asp Ser Ser Met Glu Glu
                565                 570                 575

Ile Phe Gln His Leu Met Ala Tyr His Gln Glu Ala Ser Lys Arg Ile
            580                 585                 590

Ser Ser His Ile Pro Leu Ile Ile Gln Phe Phe Met Leu Gln Thr Tyr
            595                 600                 605

Gly Gln Gln Leu Gln Lys Ala Met Leu Gln Leu Leu Gln Asp Lys Asp
        610                 615                 620

Thr Tyr Ser Trp Leu Leu Lys Glu Arg Ser Asp Thr Ser Asp Lys Arg
625                 630                 635                 640

Lys Phe Leu Lys Glu Arg Leu Ala Arg Leu Thr Gln Ala Arg Arg Arg
                645                 650                 655

Leu Ala Gln Phe Pro Gly
                660
```

```
<210>   4
<211>   335
<212>   PRT
<213>   Homo sapiens

<400>   4
```

```
Met Ala Ala Gly Ala Ala Glu Ala Ala Val Ala Ala Val Glu Glu Val
1               5                   10                  15

Gly Ser Ala Gly Gln Phe Glu Glu Leu Leu Arg Leu Lys Ala Lys Ser
            20                  25                  30
```

Leu Leu Val Val His Phe Trp Ala Pro Trp Ala Pro Gln Cys Ala Gln
35              40              45

Met Asn Glu Val Met Ala Glu Leu Ala Lys Glu Leu Pro Gln Val Ser
50              55              60

Phe Val Lys Leu Glu Ala Glu Gly Val Pro Glu Val Ser Glu Lys Tyr
65              70              75              80

Glu Ile Ser Ser Val Pro Thr Phe Leu Phe Phe Lys Asn Ser Gln Lys
85              90              95

Ile Asp Arg Leu Asp Gly Ala His Ala Pro Glu Leu Thr Lys Lys Val
100             105             110

Gln Arg His Ala Ser Ser Gly Ser Phe Leu Pro Ser Ala Asn Glu His
115             120             125

Leu Lys Glu Asp Leu Asn Leu Arg Leu Lys Lys Leu Thr His Ala Ala
130             135             140

Pro Cys Met Leu Phe Met Lys Gly Thr Pro Gln Glu Pro Arg Cys Gly
145             150             155             160

Phe Ser Lys Gln Met Val Glu Ile Leu His Lys His Asn Ile Gln Phe
165             170             175

Ser Ser Phe Asp Ile Phe Ser Asp Glu Glu Val Arg Gln Gly Leu Lys
180             185             190

Ala Tyr Ser Ser Trp Pro Thr Tyr Pro Gln Leu Tyr Val Ser Gly Glu
195             200             205

Leu Ile Gly Gly Leu Asp Ile Ile Lys Glu Leu Glu Ala Ser Glu Glu
210             215             220

Leu Asp Thr Ile Cys Pro Lys Ala Pro Lys Leu Glu Glu Arg Leu Lys
225             230             235             240

Val Leu Thr Asn Lys Ala Ser Val Met Leu Phe Met Lys Gly Asn Lys
245             250             255

Gln Glu Ala Lys Cys Gly Phe Ser Lys Gln Ile Leu Glu Ile Leu Asn
260             265             270

Ser Thr Gly Val Glu Tyr Glu Thr Phe Asp Ile Leu Glu Asp Glu Glu
275             280             285

```
Val Arg Gln Gly Leu Lys Ala Tyr Ser Asn Trp Pro Thr Tyr Pro Gln
    290             295             300

Leu Tyr Val Lys Gly Glu Leu Val Gly Gly Leu Asp Ile Val Lys Glu
    305             310             315             320

Leu Lys Glu Asn Gly Glu Leu Leu Pro Ile Leu Arg Gly Glu Asn
                325             330             335


<210>  5
<211>  281
<212>  PRT
<213>  Homo sapiens

<400>  5

Met Ala Val Asn Val Tyr Ser Thr Ser Val Thr Ser Glu Asn Leu Ser
1               5               10              15

Arg His Asp Met Leu Ala Trp Val Asn Asp Ser Leu His Leu Asn Tyr
            20              25              30

Thr Lys Ile Glu Gln Leu Cys Ser Gly Ala Ala Tyr Cys Gln Phe Met
        35              40              45

Asp Met Leu Phe Pro Gly Cys Val His Leu Arg Lys Val Lys Phe Gln
        50              55              60

Ala Lys Leu Glu His Glu Tyr Ile His Asn Phe Lys Val Leu Gln Ala
65              70              75              80

Ala Phe Lys Lys Met Gly Val Asp Lys Ile Ile Pro Val Glu Lys Leu
                85              90              95

Val Lys Gly Lys Phe Gln Asp Asn Phe Glu Phe Ile Gln Trp Phe Lys
            100             105             110

Lys Phe Phe Asp Ala Asn Tyr Asp Gly Lys Asp Tyr Asn Pro Leu Leu
        115             120             125

Ala Arg Gln Gly Gln Asp Val Ala Pro Pro Pro Asn Pro Gly Asp Gln
    130             135             140

Ile Phe Asn Lys Ser Lys Lys Leu Ile Gly Thr Ala Val Pro Gln Arg
145             150             155             160

Thr Ser Pro Thr Gly Pro Lys Asn Met Gln Thr Ser Gly Arg Leu Ser
                165             170             175
```

```
Asn Val Ala Pro Pro Cys Ile Leu Arg Lys Asn Pro Pro Ser Ala Arg
            180                 185                 190

Asn Gly Gly His Glu Thr Asp Ala Gln Ile Leu Glu Leu Asn Gln Gln
            195                 200                 205

Leu Val Asp Leu Lys Leu Thr Val Asp Gly Leu Glu Lys Glu Arg Asp
            210                 215                 220

Phe Tyr Phe Ser Lys Leu Arg Asp Ile Glu Leu Ile Cys Gln Glu His
225                 230                 235                 240

Glu Ser Glu Asn Ser Pro Val Ile Ser Gly Ile Ile Gly Ile Leu Tyr
            245                 250                 255

Ala Thr Glu Glu Gly Phe Ala Pro Pro Glu Asp Asp Glu Ile Glu Glu
            260                 265                 270

His Gln Gln Glu Asp Gln Asp Glu Tyr
            275                 280


<210>  6
<211>  244
<212>  PRT
<213>  Homo sapiens

<400>  6

Met Glu Val Thr Gly Val Ser Ala Pro Thr Val Thr Val Phe Ile Ser
1               5                   10                  15

Ser Ser Leu Asn Thr Phe Arg Ser Glu Lys Arg Tyr Ser Arg Ser Leu
            20                  25                  30

Thr Ile Ala Glu Phe Lys Cys Lys Leu Glu Leu Leu Val Gly Ser Pro
            35                  40                  45

Ala Ser Cys Met Glu Leu Glu Leu Tyr Gly Val Asp Asp Lys Phe Tyr
            50                  55                  60

Ser Lys Leu Asp Gln Glu Asp Ala Leu Leu Gly Ser Tyr Pro Val Asp
65                  70                  75                  80

Asp Gly Cys Arg Ile His Val Ile Asp His Ser Gly Ala Arg Leu Gly
                85                  90                  95

Glu Tyr Glu Asp Val Ser Arg Val Glu Lys Tyr Thr Ile Ser Gln Glu
            100                 105                 110
```

```
Ala Tyr Asp Gln Arg Gln Asp Thr Val Arg Ser Phe Leu Lys Arg Ser
        115                 120             125

Lys Leu Gly Arg Tyr Asn Glu Glu Glu Arg Ala Gln Gln Glu Ala Glu
        130                 135             140

Ala Ala Gln Arg Leu Ala Glu Glu Lys Ala Gln Ala Ser Ser Ile Pro
145                 150             155                 160

Val Gly Ser Arg Cys Glu Val Arg Ala Ala Gly Gln Ser Pro Arg Arg
                165             170             175

Gly Thr Val Met Tyr Val Gly Leu Thr Asp Phe Lys Pro Gly Tyr Trp
                180             185             190

Ile Gly Val Arg Tyr Asp Glu Pro Leu Gly Lys Asn Asp Gly Ser Val
            195                 200             205

Asn Gly Lys Arg Tyr Phe Glu Cys Gln Ala Lys Tyr Gly Ala Phe Val
        210                 215             220

Lys Pro Ala Val Val Thr Val Gly Asp Phe Pro Glu Glu Asp Tyr Gly
225                 230             235                 240

Leu Asp Glu Ile
```

```
<210>   7
<211>   453
<212>   PRT
<213>   Homo sapiens

<400>   7
```

```
Gly Ser Ala Ser Ala Pro Thr Leu Phe Pro Leu Val Ser Cys Glu Asn
1               5               10                  15

Ser Pro Ser Asp Thr Ser Ser Val Ala Val Gly Cys Leu Ala Gln Asp
            20                  25                  30

Phe Leu Pro Asp Ser Ile Thr Phe Ser Trp Lys Tyr Lys Asn Asn Ser
            35                  40                  45

Asp Ile Ser Ser Thr Arg Gly Phe Pro Ser Val Leu Arg Gly Gly Lys
        50                  55                  60

Tyr Ala Ala Thr Ser Gln Val Leu Leu Pro Ser Lys Asp Val Met Gln
65                  70                  75                  80
```

25

```
Gly Thr Asp Glu His Val Val Cys Lys Val Gln His Pro Asn Gly Asn
                85              90                      95

Lys Glu Lys Asn Val Pro Leu Pro Val Ile Ala Glu Leu Pro Pro Lys
            100             105                 110

Val Ser Val Phe Val Pro Pro Arg Asp Gly Phe Phe Gly Asn Pro Arg
        115             120                 125

Lys Ser Lys Leu Ile Cys Gln Ala Thr Gly Phe Ser Pro Arg Gln Ile
    130             135                 140

Gln Val Ser Trp Leu Arg Glu Gly Lys Gln Val Gly Ser Gly Val Thr
145             150                 155                 160

Thr Asp Gln Val Gln Ala Glu Ala Lys Glu Ser Gly Pro Thr Thr Tyr
            165             170                 175

Lys Val Thr Ser Thr Leu Thr Ile Lys Glu Ser Asp Trp Leu Ser Gln
        180             185                 190

Ser Met Phe Thr Cys Arg Val Asp His Arg Gly Leu Thr Phe Gln Gln
        195             200                 205

Asn Ala Ser Ser Met Cys Val Pro Asp Gln Asp Thr Ala Ile Arg Val
    210             215                 220

Phe Ala Ile Pro Pro Ser Phe Ala Ser Ile Phe Leu Thr Lys Ser Thr
225             230                 235                 240

Lys Leu Thr Cys Leu Val Thr Asp Leu Thr Thr Tyr Asp Ser Val Thr
            245             250                 255

Ile Ser Trp Thr Arg Gln Asn Gly Glu Ala Val Lys Thr His Thr Asn
            260             265                 270

Ile Ser Glu Ser His Pro Asn Ala Thr Phe Ser Ala Val Gly Glu Ala
        275             280                 285

Ser Ile Cys Glu Asp Asp Trp Asn Ser Gly Glu Arg Phe Thr Cys Thr
        290             295                 300

Val Thr His Thr Asp Leu Pro Ser Pro Leu Lys Gln Thr Ile Ser Arg
305             310                 315                 320

Pro Lys Gly Val Ala Leu His Arg Pro Asp Val Tyr Leu Leu Pro Pro
            325             330                 335
```

EP 2 908 134 A1

Ala Arg Glu Gln Leu Asn Leu Arg Glu Ser Ala Thr Ile Thr Cys Leu
                340                 345                 350

Val Thr Gly Phe Ser Pro Ala Asp Val Phe Val Gln Trp Met Gln Arg
                355                 360                 365

Gly Gln Pro Leu Ser Pro Glu Lys Tyr Val Thr Ser Ala Pro Met Pro
                370                 375                 380

Glu Pro Gln Ala Pro Gly Arg Tyr Phe Ala His Ser Ile Leu Thr Val
385                 390                 395                 400

Ser Glu Glu Glu Trp Asn Thr Gly Glu Thr Tyr Thr Cys Val Val Ala
                405                 410                 415

His Glu Ala Leu Pro Asn Arg Val Thr Glu Arg Thr Val Asp Lys Ser
                420                 425                 430

Thr Gly Lys Pro Thr Leu Tyr Asn Val Ser Leu Val Met Ser Asp Thr
                435                 440                 445

Ala Gly Thr Cys Tyr
        450

<210>  8
<211>  839
<212>  PRT
<213>  Homo sapiens

<400>  8

Met Ser Val Val Gly Ile Asp Leu Gly Phe Leu Asn Cys Tyr Ile Ala
1                   5                   10                  15

Val Ala Arg Ser Gly Gly Ile Glu Thr Ile Ala Asn Glu Tyr Ser Asp
                20                  25                  30

Arg Cys Thr Pro Ala Cys Ile Ser Leu Gly Ser Arg Thr Arg Ala Ile
        35                  40                  45

Gly Asn Ala Ala Lys Ser Gln Ile Val Thr Asn Val Arg Asn Thr Ile
        50                  55                  60

His Gly Phe Lys Lys Leu His Gly Arg Ser Phe Asp Asp Pro Ile Val
65                  70                  75                  80

Gln Thr Glu Arg Ile Arg Leu Pro Tyr Glu Leu Gln Lys Met Pro Asn
                85                  90                  95

27

```
Gly Ser Ala Gly Val Lys Val Arg Tyr Leu Glu Glu Glu Arg Pro Phe
            100                 105             110

Ala Ile Glu Gln Val Thr Gly Met Leu Leu Ala Lys Leu Lys Glu Thr
            115                 120             125

Ser Glu Asn Ala Leu Lys Lys Pro Val Ala Asp Cys Val Ile Ser Ile
145             150             155             160

Pro Ser Phe Phe Thr Asp Ala Glu Arg Arg Ser Val Met Ala Ala Ala
145             150             155             160

Gln Val Ala Gly Leu Asn Cys Leu Arg Leu Met Asn Glu Thr Thr Ala
            165                 170             175

Val Ala Leu Ala Tyr Gly Ile Tyr Lys Gln Asp Leu Pro Pro Leu Asp
            180                 185             190

Glu Lys Pro Arg Asn Val Val Phe Ile Asp Met Gly His Ser Ala Tyr
            195                 200             205

Gln Val Leu Val Cys Ala Phe Asn Lys Gly Lys Leu Lys Val Leu Ala
            210                 215             220

Thr Thr Phe Asp Pro Tyr Leu Gly Gly Arg Asn Phe Asp Glu Ala Leu
225             230             235             240

Val Asp Tyr Phe Cys Asp Glu Phe Lys Thr Lys Tyr Lys Ile Asn Val
            245                 250             255

Lys Glu Asn Ser Arg Ala Leu Leu Arg Leu Tyr Gln Glu Cys Glu Lys
            260                 265             270

Leu Lys Lys Leu Met Ser Ala Asn Ala Ser Asp Leu Pro Leu Asn Ile
            275                 280             285

Glu Cys Phe Met Asn Asp Leu Asp Val Ser Ser Lys Met Asn Arg Ala
            290                 295             300

Gln Phe Glu Gln Leu Cys Ala Ser Leu Leu Ala Arg Val Glu Pro Pro
305             310             315             320

Leu Lys Ala Val Met Glu Gln Ala Asn Leu Gln Arg Glu Asp Ile Ser
            325                 330             335

Ser Ile Glu Ile Val Gly Gly Ala Thr Arg Ile Pro Ala Val Lys Glu
```

```
                340                        345                           350

        Gln Ile Thr Lys Phe Phe Leu Lys Asp Ile Ser Thr Thr Leu Asn Ala
                355                    360                   365

        Asp Glu Ala Val Ala Arg Gly Cys Ala Leu Gln Cys Ala Ile Leu Ser
            370                    375                   380

        Pro Ala Phe Lys Val Arg Glu Phe Ser Ile Thr Asp Leu Val Pro Tyr
        385                    390                   395                400

        Ser Ile Thr Leu Arg Trp Lys Thr Ser Phe Glu Asp Gly Ser Gly Glu
                    405                   410                   415

        Cys Glu Val Phe Cys Lys Asn His Pro Ala Pro Phe Ser Lys Val Ile
                420                   425                   430

        Thr Phe His Lys Lys Glu Pro Phe Glu Leu Glu Ala Phe Tyr Thr Asn
                435                   440                   445

        Leu His Glu Val Pro Tyr Pro Asp Ala Arg Ile Gly Ser Phe Thr Ile
            450                   455                   460

        Gln Asn Val Phe Pro Gln Ser Asp Gly Asp Ser Ser Lys Val Lys Val
        465                   470                   475                480

        Lys Val Arg Val Asn Ile His Gly Ile Phe Ser Val Ala Ser Ala Ser
                    485                   490                   495

        Val Ile Glu Lys Gln Asn Leu Glu Gly Asp His Ser Asp Ala Pro Met
                500                   505                   510

        Glu Thr Glu Thr Ser Phe Lys Asn Glu Asn Lys Asp Asn Met Asp Lys
                515                   520                   525

        Met Gln Val Asp Gln Glu Glu Gly His Gln Lys Cys His Ala Glu His
                530                   535                   540

        Thr Pro Glu Glu Glu Ile Asp His Thr Gly Ala Lys Thr Lys Ser Ala
        545                   550                   555                560

        Val Ser Asp Lys Gln Asp Arg Leu Asn Gln Thr Leu Lys Lys Gly Lys
                    565                   570                   575

        Val Lys Ser Ile Asp Leu Pro Ile Gln Ser Ser Leu Cys Arg Gln Leu
                580                   585                   590
```

29

```
Gly Gln Asp Leu Leu Asn Ser Tyr Ile Glu Asn Glu Gly Lys Met Ile
        595                 600             605

Met Gln Asp Lys Leu Glu Lys Glu Arg Asn Asp Ala Lys Asn Ala Val
        610                 615             620

Glu Glu Tyr Val Tyr Asp Phe Arg Asp Arg Leu Gly Thr Val Tyr Glu
625                 630             635                 640

Lys Phe Ile Thr Pro Glu Asp Leu Ser Lys Leu Ser Ala Val Leu Glu
                645             650             655

Asp Thr Glu Asn Trp Leu Tyr Glu Asp Gly Glu Asp Gln Pro Lys Gln
            660             665             670

Val Tyr Val Asp Lys Leu Gln Glu Leu Lys Lys Tyr Gly Gln Pro Ile
        675             680             685

Gln Met Lys Tyr Met Glu His Glu Glu Arg Pro Lys Ala Leu Asn Asp
        690             695             700

Leu Gly Lys Lys Ile Gln Leu Val Met Lys Val Ile Glu Ala Tyr Arg
705             710             715                 720

Asn Lys Asp Glu Arg Tyr Asp His Leu Asp Pro Thr Glu Met Glu Lys
                725             730             735

Val Glu Lys Cys Ile Ser Asp Ala Met Ser Trp Leu Asn Ser Lys Met
            740             745             750

Asn Ala Gln Asn Lys Leu Ser Leu Thr Gln Asp Pro Val Val Lys Val
        755             760             765

Ser Glu Ile Val Ala Lys Ser Lys Glu Leu Asp Asn Phe Cys Asn Pro
    770             775             780

Ile Ile Tyr Lys Pro Lys Pro Lys Ala Glu Val Pro Glu Asp Lys Pro
785             790             795                 800

Lys Ala Asn Ser Glu His Asn Gly Pro Met Asp Gly Gln Ser Gly Thr
            805             810             815

Glu Thr Lys Ser Asp Ser Thr Lys Asp Ser Ser Gln His Thr Lys Ser
        820             825             830

Ser Gly Glu Met Glu Val Asp
        835
```